Europäisches Patentamt

European Patent Office

Office européen des brevets

(19)

(11) Veröffentlichungsnummer : **0 365 818 B1**

(12) **EUROPÄISCHE PATENTSCHRIFT**

(45) Veröffentlichungstag der Patentschrift :
**15.02.95 Patentblatt 95/07**

(21) Anmeldenummer : **89117109.2**

(22) Anmeldetag : **15.09.89**

(51) Int. Cl.$^6$ : **G01N 33/53,** G01N 33/94,
G01N 33/577, C12P 21/00

(54) Immunologischer Nachweis von Atrazin und Atrazinderivaten.

(30) Priorität : **19.09.88 CH 3480/88**

(43) Veröffentlichungstag der Anmeldung :
**02.05.90 Patentblatt 90/18**

(45) Bekanntmachung des Hinweises auf die
Patenterteilung :
**15.02.95 Patentblatt 95/07**

(84) Benannte Vertragsstaaten :
**AT BE CH DE ES FR GB GR IT LI LU NL SE**

(56) Entgegenhaltungen :
**EP-A- 0 300 381**
**US-A- 4 530 786**
**JOURNAL OF AGRICULTURAL & FOOD CHE-**
**MISTRY, Band 33, Nr. 3, 1985, American Che-**
**mical Society, Washington, DC (US); W.H.**
**NEWSOME, Seiten 528-530** #
**BIOLOGICAL ABSTRACTS, Band 80, Nr. 11,**
**1985, Philadelphia, PA (US); S.J. HUBER et al.,**
**Nr. 97998** #

(56) Entgegenhaltungen :
**CHEMICAL ABSTRACTS, Band 104, Nr. 11, 17.**
**März 1986, Columbus, OH (US); S.J. HUBER,**
**Seite 228, Nr. 83639e** #
**BULLETIN ON ENVIRONMENTAL CONTA-**
**MINATION & TOXICOLOGY, Band 40, 1988,**
**New York, NY (US); R.J. BUSHWAY et al.,**
**Seiten 647-654** #

(73) Patentinhaber : **CIBA-GEIGY AG**
**Klybeckstrasse 141**
**CH-4002 Basel (CH)**

(72) Erfinder : **Schlaeppi, Jean-Marc, Dr.**
**Auf der Lyss 22**
**CH-4051 Basel (CH)**
Erfinder : **Ramsteiner, Klaus, Dr.**
**Witterswilerstr. 14**
**CH-4114 Hofstetten (CH)**
Erfinder : **Föry, Werner, Dr.**
**Inzlingerstr. 11**
**CH-4125 Riehen (CH)**

(74) Vertreter : **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

Anmerkung : Innerhalb von neun Monaten nach der Bekanntmachung des Hinweises auf die Erteilung des europäischen Patents kann jedermann beim Europäischen Patentamt gegen das erteilte europä- ische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patent- übereinkommen).

EP 0 365 818 B1

## Beschreibung

Gegenstand der vorliegenden Erfindung sind monoklonale Antikörper, die sich durch eine hohe Selektivität und Affinität gegenüber Atrazin und/oder Atrazinderivaten bzw. gegenüber Hydroxyanalogen von Atrazin und/oder Atrazin derivaten auszeichnen und die sich daher in hervorragender Weise für die Verwendung in einem Immunassay zum schnellen und effektiven Nachweis von Atrazin und/oder Atrazinderivaten sowie von Hydroxyanalogen von Atrazin und/oder Atrazinderivaten eignen.

Ein weiterer Aspekt der vorliegenden Erfindung betrifft Hybridomazellllinien, die besagte monoklonale Antikörper produzieren sowie immunologische Verfahren zum Nachweis von Atrazin und/oder Atrazinderivaten sowie von Hydroxyanalogen von Atrazin und/oder Atrazinderivaten, unter Verwendung besagter monoklonaler Antikörper und die im Rahmen dieser Nachweisverfahren verwendbaren Testkits.

Der Einsatz synthetischer Herbizide für die Zwecke des Pflanzenschutzes und die damit in Zusammenhang stehenden Umweltbelastungen sind in jüngster Zeit immer häufiger in den Mittelpunkt der öffentlichen Diskussion gerückt.

s-Triazine und s-Triazinderivate wie Atrazin, Simazin oder Propazin werden seit der Entdeckung ihrer herbiziden Potenz in den 50iger Jahren in grossem Masstab für die Zwecke des Pflanzenschutzes eingesetzt, insbesondere zur Bekämpfung einjähriger, breitblättriger Unkräuter sowie verschiedener Wildgräser in Maiskulturen.

Vorsichtige Schätzungen gehen davon aus, dass gegenwärtig Maisfelder in einer Grössenordnung von 45 Millionen bis zu 100 Millionen Hektar mit s-Triazinen oder s-Triazinderivaten behandelt werden.

Es ist seit langem bekannt, dass s-Triazine, wie z.B. Atrazin, Simazin oder Propazin im Boden und anderen Biotopen nur relativ langsam abgebaut werden.

Diese Persistenz der s-Triazine im Boden ist auch für den Landwirt von grosser Bedeutung. Bei der Verwendung von Atrazin in Aufwandmengen, wie sie z.B. in Maiskulturen notwendig sind, können bereits innerhalb einer Fruchtfolge Probleme bei atrazinempfindlichen Folgekulturen auftreten, sodass der Landwirt unter Umständen gezwungen ist, eine aufwendige Bodenanalyse im Labor durchführen zu lassen. Die dort angewendeten Analyse-Verfahren sind äusserst zeit-und kostenintensiv, da sie an hochspezialisierte und aufwendige Apparaturen gebunden sind.

Aus den genannten Gründen muss es daher als eine vordringliche Aufgabe angesehen werden die bestehenden Nachweisverfahren für synthetische Herbizide, insbesondere auch für s-Triazine und s-Triazinderivate sowie für deren Abbauprodukte, zu verbessern, d.h. kostengünstigere, effizientere und leichter handhabbare Verfahren zu entwickeln, die auch ausserhalb des Labors unter Feldbedingungen angewendet werden können und die beispielsweise dem Landwirt schnell und zuverlässig Informationen darüber liefern, ob und in welchen Konzentrationen ein bestimmter Wirkstoff oder Metabolit z.B. im Boden oder Wasser vorliegt.

Von besonderer Bedeutung ist in diesem Zusammenhang die Möglichkeit zur Differenzierung des aktiven Wirkstoffs gegenüber seinen inaktiven Abbauprodukten, da nur so eine quantitative Bestimmung des tatsächlich noch im Boden vorhandenen Wirkstoffanteils möglich ist.

Der Nachweis von Atrazin und dessen Hauptabbauproduktes Hydroxyatrazin in Boden- und Wasserproben erfolgte früher in erster Linie mittels TLC (Dünnschicht-Chromatographie) sowie mit Hilfe spektroskopischer Methoden. Heute werden dagegen in der Hauptsache HPLC ('High Pressure Liquid Chromatography') (Ramsteiner und Hörmann, 1979) und GC- (Gas Chromatographie) (Ramsteiner K, 1985) Verfahren verwendet.

All die genannten Verfahren zum Nachweis synthetischer Herbizide sind jedoch mit zahlreichen Nachteilen verbunden. So müssen beispielsweise für die Bestimmung von Atrazin oder Hydroxyatrazin in Bodenproben mittels HPLC vor der Durchführung der eigentlichen chromatographischen Analyse aufwendige Reinigungs- und Aufkonzentrierungsschritte zwischengeschaltet werden.

Ein weiterer Nachteil der HPLC-Verfahren ist durch die Tatsache gegeben, dass dort photometrische Detektoren verwendet werden, die relativ unspezifisch sind. Neben der massenspektroskopischen Detektion beruhen die chromatographischen Analysen auf der Bestimmung von Retentionszeiten des jeweiligen Systems. Diese Werte sind aber relativ und damit nicht strukturspezifisch.

Die gaschromatographische Analyse des Hydroxyatrazins in Verbindung mit einer massenspektroskopischen Bestimmung ist aufgrund der geringen Flüchtigkeit dieser Verbindung nur mit erheblichem Aufwand durchführbar.

Um diese zuvor beschriebenen Nachteile der etablierten analytischen Verfahren zu vermeiden, hat man in jüngster Zeit versucht auch für den Agrarsektor immunologische Verfahren zu entwickeln, - wie sie in der klinischen Diagnostik zum Nachweis der verschiedensten Antigene bereits routinemässig eingesetzt werden - , insbesondere für die quantitative und qualitative Bestimmung von Agrarchemikalien in Boden-, Wasser- oder Luftproben.

2

So hat man beispielsweise mittlerweile begonnen immunologische Verfahren für den Nachweis bestimmter Herbizide, wie 2,4-Dichlorphenoxyessigsäure (Fleeker, 1986) oder Chlorsulfuron (Kelley et al, 1985) sowie verschiedener Pestizide, wie Diflubenzuron (Wie und Hammock, 1982), Metalaxyl (Newsome, 1985) oder Parathion (Ercegovich et al, 1981) zu entwickeln.

Auch für den immunologischen Nachweis von Atrazin ist bereits ein Verfahren beschrieben (US-P 4,530,786), das jedoch, wie auch die zuvor genannten Methoden, auf der Verwendung polyklonaler Antiseren beruht, die aus Tieren gewonnen werden, welche zuvor mit einem entsprechenden Antigen immunisiert wurden.

Polyklonale Antiseren sind in ihrer Zusammensetzung sehr heterogen, d.h. sie enthalten eine Vielzahl verschiedener Antikörper, die mit unterschiedlichen Epitopen des jeweiligen Antigens reagieren. Diese heterogene Zusammensetzung polyklonaler Antiseren kommt dadurch zustande, dass bei der Immunisierung eines Versuchstieres mit einem bestimmten Antigen immer mehrere Antikörper-produzierende Zellklone gleichzeitig stimuliert werden, von denen jeder ein anderes Epitop auf dem Antigenmolekül erkennt und daher von den stimulierten Zellklonen unterschiedliche Antikörper produziert werden.

Aus diesem Grund sind Immunseren immunisierter Tiere immer polyklonal und somit heterogen, sowohl was ihre Spezifität und als auch ihre Zugehörigkeit zu den einzelnen Klassen von Immunglobulinen angeht.

Diese Heterogenität in der Zusammensetzung polyklonaler Antiseren kann daher dazu führen, dass strukturell nahe verwandte Verbindungen, wie beispielsweise das Atrazin und dessen Hauptabbauprodukt Hydroxyatrazin bei der Verwendung polyklonaler Antikörper im Rahmen eines Immunassays nicht in ausreichendem Masse differenziert werden können.

Die Aufgabe, die es im Rahmen dieser Erfindung zu lösen galt, betraf dagegen in erster Linie die Bereitstellung eines einfach handhabbaren, effektiven und in hohem Masse selektiven Immunassays für einen schnellen und zuverlässigen Nachweis von Atrazin und/oder Atrazinderivaten sowie von inaktiven Atrazinabbauprodukten, insbesondere aber von Hydroxyatrazin und/oder Hydroxyatrazinderivaten, die eine Differenzierung von Atrazin und/oder Atrazinderivaten gegenüber ihren Abbauprodukten ermöglichen.

Diese Aufgabe konnte jetzt im Rahmen der vorliegenden Erfindung überraschenderweise gelöst werden und zwar durch die Bereitstellung von monoklonalen Antikörpern mit hoher Spezifität und Affinität für Atrazin und/oder Atrazinderivate sowie durch die Bereitstellung von monoklonalen Antikörpern mit hoher Spezifität und Affinität für die Hydroxyanalogen von Atrazin und/oder Atrazinderivaten, wie z.B. Hydroxyatrazin, Hydroxysimazin, Hydroxypropazin etc., unter Verwendung der an sich bekannten Hybridoma/monoklonalen Antikörper-Technologie.

Die Verwendung hybrider somatischer Zellinien (Hybridomas) als Quelle für Antikörper gegen ganz bestimmte Antigene geht auf die Arbeiten von Köhler und Milstein (Nature 256: 495-97, 1975) zurück.

Die Antikörper, die sich mit dem dort beschriebenen Verfahren gewinnen lassen, unterscheiden sich sehr stark von denjenigen, die man aus Antiseren konventionell immunisierter Tiere erhält.

Das Prinzip der Hybridoma/monoklonalen Antikörper-Technologie gründet sich auf die Beobachtung, dass beim Verschmelzen zweier somatischer Zellen die resultierende Hybridzelle charakteristische Merkmale beider Elterntypen aufweist.

Im Falle der monoklonalen Antikörperproduktion stammt die Fähigkeit zur Synthese des spezifischen Antikörpers von einer immunkompetenten B-Zelle (gewöhnlich einer Milzzelle), die einem zuvor immunisierten Donor-Tier entnommen wurde, während die Fähigkeit zur kontinuierlichen Zellteilung in Kultur durch den anderen Fusionspartner, eine Tumorzellinie (oft ein Myeloma) beigesteuert wird.

Jede dieser Hybrid-Zellinien synthetisiert ein homogenes Immunglobulin, das nur einen einzigen Vertreter aus der grossen Anzahl möglicher Antikörper repräsentiert, die von einem Tier als Antwort auf ein Antigen in vivo synthetisiert werden kann.

Da jeder Immunglobulin-produzierende Klon durch einen einzigen Typ von Antikörpern charakterisiert wird, hat sich die Bezeichnung monoklonale Antikörper eingebürgert.

Die Vorteile monoklonaler gegenüber polyklonalen Antikörpern sind zahlreich:

a) monokolonale Antikörper können in grosser Anzahl und in hohem Reinheitsgrad erhalten werden,

b) die Herstellung monoklonaler Antikörper ist homogen im Hinblick auf die Antigen-Reaktivität und ändert sich auch nicht im Verlaufe der Zeit,

c) monoklonale Antikörper produziernde Hybridomas können über Jahre und Jahrzehnte hinweg aufbewahrt werden, ohne dabei ihre spezifischen Eigenschaften, i.e. die Produktion spezifischer monoklonaler Antikörper, zu verlieren,

d) monoklonale Antikörper sind für die Verwendung als Standardreagentien besser geeignet als polyklonale Antiseren, da letztere negativ beeinflusst werden durch eine grosse Variationsbreite beispielsweise im Hinblick auf

α) die Blutentnahme immunisierter Tiere zur Gewinnung des Antiserums,

3

β) eine ständige Verfügbarkeit von Material für zusätzliche Immunisierungen,

γ) die begrenzte Lebenszeit der Donortiere.

Monoklonale Antikörper, die mittlerweile gegen eine Vielzahl von Antigenen hergestellt wurden, sind in erster Linie in der medizinischen Diagnostik gut etabliert und aus dieser nicht mehr wegzudenken.

Die Verwendung monoklonaler Antikörper auf dem Agrarsektor beschränkte sich dagegen bisher in erster Linie auf das Screening von Pflanzenkrankheiten und die Entwicklung von Vakzinen für Nutztiere.

Im Zusammenhang mit Pflanzenkrankheiten wurden beispielsweise von Hsu H.T., et al. (ASM News, 50(3): 91-101, 1984) insgesamt 18 Spezies von Pflanzenviren aufgelistet, gegen die monoklonale Antikörper entwickelt wurden, darunter "Carnation Etched Ring Virus", "Potato Leaf Roll Virus", "Southern Bean Mosaik Virus", Tabak Mosaik Virus, "Tomato Ring Spot Virus" sowie "Tulip Breaking Virus".

Im Rahmen der vorliegenden Erfirdung ist es jetzt erstmals gelungen unter Anwendung der an sich bekannten und zuvor kurz beschriebenen Hybridoma/monoklonalen Antikörper-Technologie sowohl monokonale Antikörper mit hoher Spezifität und Affinität für Atrazin und/oder Atrazinderivate als auch monoklonale Antikörper mit hoher Spezifität und Affinität für inaktive Hydroxy-Abbauprodukte von Atrazin und/oder Atrazinderivaten zur Verfügung zu stellen, die aufgrund ihrer hohen Spezifität und der dadurch bedingten geringen gegenseitigen Kreuzreaktivität in hervorragender Weise für eine Verwendung in einem Immunassay zum schnellen und zuverlässigen Nachweis von Atrazin und/oder Atrazinderivaten auf der einen sowie von deren inaktiven Hydroxy-Abbauprodukten auf der anderen Seite geeignet sind und die damit auch für eine Differenzierung von Atrazin und/oder Atrazinderivaten gegenüber ihren inaktiven Metaboliten verwendet werden können.

Die vorliegende Erfindung betrifft somit in erster Linie monoklonale Antikörper und deren Derivate, die eine hohe Spezifität und Affinität gegenüber Atrazin aufweisen und die eine ausgeprägte Kreuzreaktivität mit strukturell ähnlichen Atrazinderivaten ausgewählt aus der Gruppe bestehend aus Propazin, Prometryn und Prometon zeigen, die aber im wesentlichen keine Kreuzreaktivität mit anderen Atrazinderivaten, insbesondere aber mit Ametryn und Atraton, oder mit Hydroxyanalogen von Atrazin und/oder Atrazinderivaten, ausgewählt aus der Gruppe bestehend aus Hydroxyatrazin, Hydroxypropazin, Hydroxysimazin, Hydroxydesmetryn und Hydroxyterbuthylazin, aufweisen.

Besonders bevorzugt im Rahmen dieser Erfindung sind monoklonale Antikörper und deren Derivate, die eine hohe Spezifität und Affinität gegenüber Atrazin aufweisen und die eine Kreuzreaktivität mit Propazin von mehr als 80% zeigen, die aber im wesentlichen keine Kreuzreaktivität mit anderen Atrazinderivaten, insbesondere aber mit Ametryn und Atraton, oder mit Hydroxyanalogen von Atrazin und/oder Atrazinderivaten, ausgewählt aus der Gruppe bestehend aus Hydroxyatrazin, Hydroxypropazin, Hydroxysimazin, Hydroxydesmetryn und Hydroxyterbuthylazin, aufweisen.

Ein weiterer Gegenstand dieser Anmeldung betrifft monoklonale Antikörper und deren Derivate, die eine hohe Spezifität und Affinität gegenüber Hydroxyatrazin aufweisen und die im wesentlichen keine Kreuzreaktivität mit Atrazin und/oder Atrazinderivaten, ausgewählt aus der Gruppe bestehend aus Propazin, Ametryn und Atraton, zeigen.

Ebenfalls umfasst von der vorliegenden Erfindung sind monoklonale Antikörper und deren Derivate, die eine hohe Spezifität und Affinität gegenüber Hydroxyatrazin aufweisen und die eine stark ausgeprägte Kreuzreaktivität mit Hydroxypropazin zeigen, die vorzugsweise bis zu 100 % beträgt, die aber im wesentlichen keine Kreuzreaktivität mit Hydroxyanalogen anderer Atrazinderivate, ausgewählt aus der Gruppe bestehend aus Hydroxysimazin, Hydroxydesmetryn und Hydroxyterbutylazin, oder mit Atrazin und/oder Atrazinderivaten, ausgewählt aus der Gruppe bestehend aus Propazin, Ametryn und Atraton, zeigen

Weiterhin umfasst sind monoklonale Antikörper und deren Derivate, die eine hohe Spezifität und Affinität gegenüber Hydroxyatrazin aufweisen und die eine Kreuzreaktivität mit Hydroxyanalogen von Atrazinderivaten, ausgewählt aus der Gruppe bestehend aus Hydroxypropazin, Hydroxysimazin und Hydroxydesmetryn zeigen, die vorzugsweise wenigstens 40 % beträgt, die aber im wesentlichen keine Kreuzreaktivität mit Atrazin und/oder Atrazinderivaten, ausgewählt aus der Gruppe bestehend aus Propazin, Ametryn und Atraton, zeigen.

Unter Derivaten monoklonaler Antikörper sind im Rahmen der vorliegenden Erfindung z.B. Antikörperfragmente zu verstehen, die noch die hohe Spezifität und Affinität für die antigenen Determinanten von Atrazin und/oder Atrazinderivaten bzw. Hydroxyatrazin und/oder Hydroxyanalogen von Atrazinderivaten besitzen, desweiteren radioaktiv markierte monoklonale Antikörper, die beispielsweise mit radioaktivem Jod ($^{125}$I, $^{131}$I) Kohlenstoff ($^{14}$C), Schwefel ($^{35}$S), Tritium ($^{3}$H) oder ähnlichem markiert sind, Konjugate monoklonaler Antikörper mit Biotin oder Avidin, mit Enzymen, wie Meerrettichperoxidase, alkalische Phosphatase, β-D-Galaktosidase, Glucoseoxidase, Glucoamylase, Carbonsäureanhydrase, Acetylcholinesterase, Lysozym, Malatdehydrogenase oder Glucose-6-phosphatdehydrogenase, desweiteren Konjugate monoklonaler Antikörper mit biolumineszierenden (z.B. Luciferase), chemolumineszierenden (z.B. Acridiniumester) oder fluoreszierenden (z.B. Phycobiliproteine) Agentien. Ebenso umfasst von der vorliegenden Anmeldung sind bispezifische sowie sog. "cross-linked" Antikörper. Diese beispielhafte Aufzählung möglicher Antikörperderivate dient lediglich der Illustration der vorliegenden Erfindung und soll den Erfindungsgegenstand in keiner Weise limitieren.

Unter der Bezeichnung "im wesentlichen keine Kreuzreaktivität" soll im Rahmen dieser Erfindung verstanden werden, dass die Reaktivität der für Atrazin bzw. Hydroxyatrazin spezifischen monoklonalen Antikörper mit unspezifischen Epitopen anderer Verbindungen, insbesondere strukturell verwandter Verbindungen weniger als 20 %, vorzugsweise aber weniger als 5 % und ganz besonders bevorzugt weniger als 1 % beträgt.

Der prozentuale Anteil der Kreuzreaktivität soll im Rahmen dieser Erfindung definitionsgemäss durch die folgende Beziehung wiedergegeben werden:

(Atrazin/Hydroxyatrazinkonzentration für 50 % Hemmung/Konzentration der s-Triazin-/Hydroxytriazinanalogen für 50 % Hemmung) x 100.

Eine 50 %ige Hemmung kann beispielsweise mit Hilfe eines kompetitiven ELISA Assays (vgl. Beispiel 8) bestimmt werden. Diese entspricht dann beispielsweise derjenigen Antigenkonzentration, die zu einer 50 %igen Hemmung der Antikörperbindung an das Träger-gebundene Antigen führt.

Ein weiterer Gegenstand der vorliegenden Erfindung betrifft Hybridomazellinien, welche die zuvor näher charakterisierten monoklonalen Antikörper synthetisieren und vorzugsweise in das umgebende Medium sezernieren.

Insbesondere betrifft die vorliegende Erfindung eine Hybridomazellinie, die einen monoklonalen Antikörper produziert, der eine hohe Spezifität und Affinität gegenüber Atrazin aufweist und der eine ausgeprägte Kreuzreaktivität mit strukturell ähnlichen Atrazinderivaten ausgewählt aus der Gruppe bestehend aus Propazin, Prometryn und Prometon zeigt, der aber im wesentlichen keine Kreuzreaktivität mit anderen Atrazinderivaten, insbesondere aber mit Ametryn und Atraton, oder mit Hydroxyanalogen von Atrazin und/oder Atrazinderivaten, ausgewählt aus der Gruppe bestehend aus Hydroxyatrazin, Hydroxypropazin, Hydroxysimazin, Hydroxydesmetryn und Hydroxyterbuthylazin, aufweist.

Besonders bevorzugt ist dabei eine Hybridomazellinie, die bei der 'European Collection of Animal Cell Cultures (ECACC) in Salisbury, UK, entsprechend den Bestimmungen des Budapester Vertrages unter der Nummer ECACC 8808/2501 hinterlegt wurde.

Ebenso umfasst ist eine Hybridomazellinie, die einen monoklonalen Antikörper produziert, der eine hohe Spezifität und Affinität gegenüber Hydroxyatrazin aufweist und der im wesentlichen keine Kreuzreaktivität mit Atrazin und/oder Atrazinderivaten, ausgewählt aus der Gruppe bestehend aus Propazin, Ametryn und Atraton, zeigt.

Ein weiterer Gegenstand dieser Erfindung betrifft eine Hybridomazellinie, die einen monoklonalen Antikörper produziert, der eine hohe Spezifität und Affinität gegenüber Hydroxyatrazin aufweist und der eine stark ausgeprägte Kreuzreaktivität mit Hydroxypropazin zeigt, der aber im wesentlichen keine Kreuzreaktivität mit Hydroxyanalogen anderer Atrazinderivate, ausgewählt aus der Gruppe bestehend aus Hydroxysimazin, Hydroxydesmetryn und Hydroxyterbutylazin, oder mit Atrazin und/oder Atrazinderivaten, ausgewählt aus der Gruppe bestehend aus Propazin, Ametryn und Atraton, zeigt.

Besonders bevorzugt ist dabei eine Hybridomazellinie, die bei der 'European Collection of Animal Cell Cultures (ECACC) in Salisbury, UK, entsprechend den Bestimmungen des Budapester Vertrages unter der Nummer ECACC 8808/2503 hinterlegt wurde.

Weiterhin umfasst ist eine Hybridomazellinie, die einen monoklonalen Antikörper produziert, der eine hohe Spezifität und Affinität gegenüber Hydroxyatrazin aufweist und der eine Kreuzreaktivität mit Hydroxyanalogen von Atrazinderivaten, ausgewählt aus der Gruppe bestehend aus Hydroxypropazin, Hydroxysimazin und Hydroxydesmetryn, zeigt, der aber im wesentlichen keine Kreuzreaktivität mit Atrazin und/oder Atrazinderivaten, ausgewählt aus der Gruppe bestehend aus Propazin, Ametryn und Atraton, zeigt.

Besonders bevorzugt ist dabei eine Hybridomazellinie, die bei der 'European Collection of Animal Cell Cultures (ECACC) in Salisbury, UK, entsprechend den Bestimmungen des Budapester Vertrages unter der Nummer ECACC 8808/2502 hinterlegt wurde.

Ebenso umfasst von der vorliegenden Erfindung sind Varianten und Mutanten der zuvor näher charakterisierten Hybridomazellinien, die spontan entstehen oder aber artifiziell mit Hilfe bekannter Verfahren hergestellt werden können und die noch die charakteristischen Eigenschaften des Ausgangsmaterials aufweisen, d.h. die noch in der Lage sind die erfindungsgemässen Antikörper oder Derivate davon zu produzieren und ins umgebende Medium zu sezernieren.

Ebenso umfasst von der vorliegenden Erfindung sind Verfahren zur Herstellung besagter Hybridomazellinien sowie Verfahren zur Herstellung besagter monoklonaler Antikörper.

Unter Klonen und Subklonen von Hybridomazellinien sind Hybridomas zu verstehen, die durch wiederholtes Klonieren aus dem Ausgangsklon hervorgehen und die noch die erfindungswesentlichen Merkmale des Ausgangsklons aufweisen.

Ein weiterer Gegenstand dieser Erfindung betrifft ein Verfahren zum immunologischen Nachweis von Atrazin und/oder Atrazinderivaten, z.B. in Boden-, Wasser- oder Luftproben sowie in biologischem Material, wie z.B. in pflanzlichen oder tierischen Extrakten, unter Verwendung der erfindungsgemässen monoklonalen Antikörper.

Weiterhin betrifft die vorliegende Erfindung ein Verfahren zum immunologischen Nachweis von Hydroxyatrazin und/oder Hydroxyanalogen von Atrazinderivaten, z.B. in Boden-, Wasser- oder Luftproben sowie in biologischem Material, wie z.B. in pflanzlichen oder tierischen Extrakten, unter Verwendung der erfindungsgemässen monoklonalen Antikörper.

Ganz besonders bevorzugt ist ein Verfahren zum Nachweis von Hydroxyatrazin.

Ebenso ein Bestandteil der vorliegenden Erfindung sind Mittel für die qualitative und quantitative Bestimmung von Atrazin und/oder Atrazinderivaten bzw. von Hydroxyanalogen von Atrazin und/oder Atrazinderivaten, wie z.B. Hydroxyatrazin, Hydroxypropazin, Hydroxysimazin etc. in Form gebrauchsfertiger Test-Kits, welche mindestens einen der erfindungsgemässen monoklonalen Antikörper als Reagenz enthalten und die für die Verwendung unter Feldbedingungen für einem schnellen und zuverlässigen Nachweis von Atrazin und/oder Atrazinderivaten, sowie von Hydroxyatrazin und/oder Hydroxyanalogen von Atrazinderivaten, geeignet sind.

Die Herstellung der erfindungsgemässen monoklonalen Antikörper erfolgt mit Hilfe an sich bekannter Verfahren, die im wesentlichen auf den von Köhler und Milstein (Nature, 256: 495-497, 1975) entwickelten Methoden beruhen.

Da es sich bei den zu analysierenden Zielsubstanzen Atrazin und Hydroxyatrazin, für die spezifische monoklonale Antikörper entwickelt werden sollen, um relativ kleine und einfache Moleküle handelt, die nach der Applikation in ein Versuchstier alleine nicht in der Lage sind dort eine entsprechende Immunantwort auszulösen, müssen vor der eigentlichen Immunisierung zunächst vorbereitende Massnahmen getroffen werden.

Man bezeichnet solche Verbindungen, die aufgrund ihrer Grösse und einfachen Strukturierung nicht zur Induktion einer Immunreaktion in der Lage sind, als Haptene oder inkomplette Antigene und stellt sie somit den kompletten Antigenen (= Immunogene) gegenüber, die sowohl als Antigen wirken als auch eine Immunantwort zu induzieren vermögen. Solche Haptenmoleküle können an hochmolekulare Verbindungen (Trägermoleküle) konjugiert werden, wodurch sie in ihren Eigenschaften kompletten Antigenen vergleichbar werden, d.h. sie besitzen jetzt die Fähigkeit zur Auslösung einer Immunantwort.

Einige der im Verlaufe der Immunisierungsreaktion gebildeten Antikörper sind dann in der Lage mit spezifischen Epitopen auf dem Haptenmolekül zu reagieren, unabhängig davon, ob das Haptenmolekül alleine oder aber nach wie vor gekoppelt an das Carriermolekül vorliegt.

Unter der im folgenden häufig verwendeten Bezeichnung Hapten sollen im Rahmen dieser Erfindung in erster Linie die für die Immunisierung verwendeten Atrazin- und/oder Hydroxyatrazin-Moleküle verstanden werden.

Im Rahmen dieser Erfindung werden daher die als Haptene wirkenden Atrazin- und Hydroxyatrazinmoleküle vor der Immunisierung von Versuchstieren an einen hochmolekularen Träger ('Carrier') gekoppelt, der geeignet ist besagten Atrazin- und Hydroxyatrazinmolekülen eine komplette antigene Wirksamkeit zu verleihen.

Unter geeigneten Carrier-Molekülen sind im Rahmen dieser Erfindung in erster Linie makromolekulare Verbindungen zu verstehen, die frei zugängliche reaktive Gruppen für die Kupplungsreaktion mit dem Hapten aufweisen und die in der Lage sind, durch Kupplung an das Hapten, diesem eine immunogene Potenz zu verleihen oder aber deren bereits vorhandene Immunogenizität zu verstärken.

Besonders bevorzugt im Rahmen dieser Erfindung sind makromolekulare Verbindungen, die frei zugängliche reaktive Aminogruppen enthalten.

Ganz besonders bevorzugt für die erfindungsgemässe Verwendung als Carriermolekül sind lysinreiche Proteine mit einem Molekulargewicht zwischen 10'000 und 1'500'000, wie z.B. "Bovine Serum Albumin" (BSA: MG 66'200), Humanes Serum Albumin (HSA,; MG 58'000) oder "Keyhole Limpet Protein" (KLH; MG 1'000'000), die kommerziell erhältlich sind und somit in beliebiger Menge zur Verfügung stehen.

Selbstverständlich können im Rahmen der vorliegenden Erfindung auch andere makromolekulare Verbindungen als Carrier-Moleküle verwendet werden, sofern sie die oben genannten Voraussetzungen erfüllen, wie z.B. "Porcine Thyroglobulin", B2 Microglobulin, Hemocyanin, Immunglobuline, Toxine (Cholera-, Tetanus-, Diphtheria-Toxin u.a.), Polysaccharide, Lipopolysaccharide, natürliche oder synthetische Polyadenyl- und Polyuridylsäuren, Polyalanyl- und Polylysin-Polypeptide oder Zellmembrankomponenten, wie beispielsweise Formalin- oder Glutaraldehyd-behandelte Erythrozytenzellmembranen.

Ebenso geeignet für die Verwendung als Carriermolekül in dem erfindungsgemässen Verfahren ist beispielsweise die gereinigte IgG Fraktion gegen Maus IgG (H+L) aus Kaninchen gemäss dem bei H Kawamura und JA Berzofsky (J. Immunol., 136: 58, 1986) beschriebenen Verfahren.

Die Konjugation des Haptens an das Trägermolekül kann entweder direkt oder aber vorzugsweise über ein Brückenglied erfolgen, welches gegebenenfalls zunächst an das Haptenmolekül angelagert wird.

Die Kupplung der zu analysierenden Substanz an das Trägermolekül muss dabei in einer Weise erfolgen, dass die relevanten Strukturelemente der Zielsubstanzen frei zugänglich bleiben und somit in der Lage sind eine spezifische Immunantwort auszulösen, d.h. die Bildung spezifischer Antikörper zu induzieren.

Als Brückenglieder für eine Konjugation des Haptens (Atrazin und/oder Atrazinderivate sowie die Hydroxyanalogen von Atrazin und/oder Atrazinderivaten) an das Carriermolekül kommen in erster Linie Verbindungen in Betracht, die mindestens eine oder aber mehrere reaktive Gruppen enthalten, welche in der Lage sind mit den frei zugänglichen reaktiven Gruppen des Carrier-Moleküls in Wechselwirkung zu treten.

Besonders bevorzugt im Rahmen dieser Erfindung ist die Verwendung von Brückengliedern die zwischen 3 und 10 Brücken-C-atome umfassen und die als reaktive Gruppe(n) eine oder mehrere reaktive Gruppen, wie z.B. Amino-, Carboxyl- oder SH-Gruppe(n) besitzen.

Diese reaktiven Gruppen können mit Hilfe an sich bekannter Verfahren mit den reaktiven Gruppen des Hapten- und Carrier-Moleküls zur Reaktion gebracht werden, unter Ausbildung eines Hapten-Carrier-Konjugates.

So ist es beispielsweise möglich ein Brückenglied über eine reaktive Aminogruppe mit Hilfe von Dialdehyden (z.B. Glutardialdehyd) an eine der freien Aminogruppen des Carrier-Moleküls zu binden.

Besitzt das Brückenglied eine reaktive SH-Gruppe, so kann die Konjugation des Haptens an das Carrier-Molekül durch eine Oxidation mit freien SH-Gruppen des Carriers durchgeführt werden.

Besonders bevorzugt im Rahmen dieser Erfindung ist die Verwendung von Brückengliedern mit einer Carboxylgruppe, die mit Hilfe von wasserbindenden Mitteln, wie beispielsweise einem Carbodiimid, vorzugsweise N,N′-Dicyclohexylcarbodiimid, mit einer freien Aminogruppe des Carriermoleküls verknüpft werden kann.

In einer spezifischen Ausführungsform der vorliegenden Erfindung geht man von Atrazin, Hydroxyatrazin oder einem ihrer Derivate, vorzugsweise von 2,6-Dichlor-4-(isopropylamino)-s-triazin aus und setzt dies mit $\delta$-Amino-valeriansäure um unter Bildung von 2-Chlor-4-(isopropylamino)-6-($\delta$-amino-valeriansäure)-s-triazin. Diese Verbindung kann dann mit Hilfe bekannter Methoden, wie z.B. durch Zugabe von wässrigen Mineralsäuren, in einem weiteren Reaktionsschritt sehr einfach in das entsprechende Hydroxy-Analoge umgewandelt werden.

Die Durchführung der eigentlichen Kupplungsreaktion erfolgt vorzugsweise mit Hilfe der aktiven Estermethode. Dabei werden die Atrazin- bzw. Hydroxyatrazinderivate zunächst in einem geeigneten Lösungsmittel solubilisiert. Als geeignete Lösungsmittel kommen insbesondere aprotische Lösungsmittel, die eine geringe Verdampfungsrate aufweisen, wie z.B. N,N-Dimethylformamid (DMF) oder Dimethylsulfoxid (DMSO) in Betracht.

Anschliessend werden die Carboxylgruppen zu einem aktiven Ester derivatisiert, indem die zuvor solubilisierten Atrazin- und/oder Hydroxyatrazinderivate z.B. mit N-Hydroxysuccinimid, N-Hydroxysulfosuccinimid, N,N′-Dicyclohexylcarbodiimid oder N,N′-Carbonyldiimidazol oder mit Derivaten dieser Verbindungen zur Reaktion gebracht werden.

Der aktive Ester wird dann aus dem Reaktionsgemisch abgetrennt und zu BSA oder KLH zugegeben. Nach einer Inkubationszeit von 0.1 bis 12 Stunden vorzugsweise von 4 bis 5 Stunden wird das Präzipitat entfernt. Der Ueberstand kann dann gegebenenfalls nach Zwischenschaltung weiterer Reinigungsschritte für die eigentliche Immunisierungsreaktion verwendet werden.

Neben der im Rahmen dieser Erfindung bevorzugten aktiven Estermethode, können auch alternative Verfahren für die Kupplung des Haptens an das Carrier-Molekül verwendet werden, wie z.B. die gemischte Anhydridmethode. Dabei wird die Carboxylgruppe des Brückengliedes unter Verwendung von Essigsäureanhydrid oder des Carbodiimidderivates 1-Ethyl-3-(3′-dimethylaminopropyl)carbodiimid an das Carrier-Molekül geknüpft.

Die Immunisierung der Donortiere erfolgt durch eine ein- bis mehrmalige Applikation der an ein hochmolekulares Trägermolekül gekoppelten Haptene. Besonders bevorzugt ist eine 2 bis 3 malige Applikation, die in Abständen von 7 bis 30 Tagen insbesondere aber von 13 bis 15 Tagen erfolgt.

Die im Rahmen der vorliegenden Erfindung bevorzugte Applikationsform ist die Injektion, die sowohl intravenös, intraperitoneal oder subcutan erfolgen kann.

Bevorzugt ist eine Kombination von subcutaner und intraperitonealer Injektion.

Nach einer Ruheperiode von 0,5 bis 4 Monaten erfolgt eine weitere einmalige Applikation des Haptenkonjugates in einer Dosierung von 100 µg bis 1000 µg.

In einem Zeitraum von 1 bis 6 Tagen nach der letzten Dosierung werden die Donortiere getötet und es wird eine Milzzellensuspension hergestellt.

Die isolierten Milzzellen werden dabei in einem geeigneten Puffer (z.B. einem BSS-Puffer) suspendiert und bis zu ihrer Fusion mit geeigneten Myeloma-Zellen in Form einer Zellsuspension aufbewahrt.

Anfänglich wurden diese Fusionen dadurch kompliziert, dass auch die Myeloma-Zellinien monoklonale Antikörper synthetisierten, so dass das Hybrid zwei Typen monoklonaler Antikörper produzierte, einen, der seinen Ursprung in der Myeloma-Zelle hatte und einen zweiten, der durch die genetische Information der immunokompetenten Zelle bestimmt wurde.

Im Rahmen der vorliegenden Erfindung werden daher vorzugsweise solche Tumorzellen verwendet, die selbst keine monoklonale Antikörper herstellen können, wie z.B. SP2/0-Ag14 (Shulman et al, 1978) oder X63-Ag8.653, wodurch die Analyse der resultierenden Fusionsprodukte sehr stark vereinfacht wird. Für den Fusionserfolg ist es vorteilhaft, wenn die Milzzellen in einem 2 bis 20fachen Ueberschuss im Verhältnis zu den Myelomzellen vorliegen.

Die Fusion von Milz- und Myelomzellen wird in einem speziellen Fusionsmedium durchgeführt, das eine Zusammensetzung aufweist, die für die beabsichtigte Zellfusion optimale Voraussetzungen liefert.

Vorzugsweise handelt es sich bei besagtem Fusionsmedium um eine Pufferlösung, die einen der üblicherweise für die Fusionierung von Zellen verwendeten Fusionspromotoren enthält, wie z.B. Sendai Viren oder andere Paramyxoviren, gegebenenfalls in UV-inaktivierter Form, Calcium-Ionen, oberflächenaktive Lipide, wie z.B. Lysolecithin oder Polyethylenglykol. Besonder bevorzugt im Rahmen dieser Erfindung ist die Verwendung von Polyethylenglykol (PEG), insbesondere von Polyethylenglykol (PEG) mit einem mittleren MG von 600 bis 6000, und in einer Konzentration von 30 % bis 60 %. Besonders bevorzugt ist eine PEG-Konzentration von 40 % - 50 %. Die optimale Fusionstemperatur beträgt zwischen 18°C und 39°C. Besonders bevorzugt ist eine Temperatur von 37°C.

Nach der erfolgten Fusion der immunkompetenten Milzzellen mit den Myeloma-Zellen werden die fusionierten Antikörper-produzierenden Hybridzellen mit Hilfe an sich bekannter Verfahren selektioniert.

Für das Auslesen erfolgreicher Fusionsereignisse (Hybridzellen) aus den 2 Typen von Elternzellen existieren verschiedene Möglichkeiten. Routinemässig werden eine Million und mehr Zellen von jedem Elterntyp in dem Fusionsprotokoll verwendet. Da die Fusion nicht mit einer 100 %igen Frequenz erfolgt, kann es zu einem schwierigen Unterfangen werden, die Fusionsprodukte von der grossen Ueberzahl nicht fusionierter oder mit sich selbst fusionierter Elternzellen abzutrennen.

Wie bereits zuvor erwähnt, entstehen die Hybridomazellen durch Fusion kurzlebiger Antikörper produzierender (Milz) B-Zellen sowie langlebiger Myeloma Zellen.

Das gewünschte Resultat ist eine langlebige Zellinie, die Antikörper produziert. Da die Milzzellen nur eine begrenzte Lebenszeit in Kultur besitzen, kann man daher im Prinzip einfach abwarten, bis alle nicht fusionierten sowie alle mit sich selbst fusionierten Milzzellen abgestorben sind. Dann bleibt jedoch immer noch die Aufgabe bestehen, die langlebigen Antikörper-produzierenden Zellen von den langlebigen nicht Antikörper-produzierenden Zellen abzutrennen.

Ein gängiges Selektionssystem basiert auf der Verfügbarkeit oder Nichtverfügbarkeit des Enzyms Hypoxanthinguaninphosphoribosyltransferase (HGPRT) Dieses Enzym ist Bestandteil des Purin "Salvage Pathway" in Säugerzellen.

Diese Zellen sind darüberhinaus auch in der Lage Purine de novo zu synthetisieren.

Unter normalen Umständen arbeiten wahrscheinlich beide Synthese-Wege bis zu einem gewissen Grade parallel.

Falls eine Zelle jedoch kein HGPRT besitzt, ist der "Salvage Pathway" blockiert und die Purine müssen aus nicht-Purin Material hergestellt werden.

Für die Selektion HGPRT-negativer Myelomazellen verwendet man in der Regel sog. Purin-Antimetaboliten, wie z.B. das 8-Azaguanin, das dem Purin Guanin strukturell sehr ähnlich ist und dieses daher in einigen seiner normalen Reaktionen ersetzen kann.

Azaguanin wird in die DNA eingebaut, was zu einer Beeinträchtigung des normalen Wachstumsverhaltens und schliesslich zum Zelltod führt. Da Azaguanin über den "Salvage Pathway" ersetzt werden muss, können alle diejenigen Zellen, die keine HGPRT Aktivität besitzen, Azaguanin nicht verwerten und daher in dessen Gegenwart wachsen.

Ein selektives System, das mit demselben Enzym aber unter umgekehrten Vorzeichen arbeitet, indem in diesem Fall HGPRT positive Zellen selektiert werden, ist bei J.W. Littlefield (1964) beschrieben.

Dieses Selektionssystem basiert auf der Verwendung des sogenannten HAT-Mediums, das u.a. Hypoxanthin, Aminopterin und Thymidin (HAT-Medium) als Bestandteile enthält. Aminopterin ist ein Antimetabolit, der die de novo Purin-Synthese sowie die Methylierung von Desoxyuridylat zu Thymidylat hemmt.

Hypoxanthin kann als Hilfspurin fungieren für den Fall, dass Aminopterin die de novo Purin-Synthese blockiert, während Thymidin die Notwendigkeit einer Methylierung von Desoxyuridylat überflüssig macht.

Daher werden in Gegenwart von Aminopterin alle HGPRT positiven Zellen proliferieren, während die HGPRT negativen Zellen absterben.

In dem Hybridsystem, das im Rahmen dieser Erfindung für die Selektion verwendet wird, sind die Myeloma-Zellen vorzugsweise resistent gegenüber Azoguanin und empfindlich gegenüber Aminopterin, d.h. sie sind HGPRT negativ. Die Antikörper-produzierenden Zellen dagegen sind HGPRT positiv.

Durch Fusion der Zellen und Kultivierung in einem HAT-Medium, können die erfolgreich miteinander fusionierten Zellen selektiert werden, da die Myeloma-Zellen, die für die Proliferation verantwortlich sind, nur in Gegenwart einer HGPRT-Aktivität wachsen können und diese Aktivität von der HGPRT-positiven Zellinie geliefert werden muss.

Die HGPRT-positiven Antikörper-produzierenden Zellinien werden in diesem Medium zwar nicht abgetötet. Sie überleben aber nur eine bestimmte Zeit, und sind nicht in der Lage zu proliferieren.

Damit wird durch Fusion der Zellen in einem HAT-Medium ein System geschaffen, in welchem zwar die Myeloma Zellen und die Antikörper produzierenden Zellen für einen Zeitraum wachsen können, der ausreicht für die Produktion von Hybrid-Zellen, in dem aber nur die Hybrid-Zellen überleben und proliferieren können.

In einer besonderen Ausführungsform der vorliegenden Erfindung werden die fusionierten Hybridzellen in Gegenwart von zuvor aus dem Peritoneum unbehandelter, nicht-immunisierter Versuchstiere isolierten Macrophagen, sogenannter "Feederzellen", kultiviert. Für die Kultivierung und die Selektion der fusionierten Hybridzellen wird die Zellsuspension in mehrere Aliquots aufgeteilt und die einzelnen Aliquots werden ständig auf die Ausbildung von Hybridzellkulturen sowie auf die Bildung von Antikörpern hin untersucht.

Besonders bevorzugt im Rahmen dieser Erfindung ist die Kultivierung der fusionierten Hybridzellen auf Mikrotiterplatten.

Dabei wird die nach der Fusion erhaltene Zellsuspension auf die einzelnen Vertiefungen einer Mikrotiterplatte verteilt und für einen Zeitraum von 7 bis 30 Tagen unter geeigneten, das Wachstum der fusionierten Hybridzellen fördernden Bedingungen (z.B. HAT/HT-Medien) kultiviert.

Die Ueberstände gewachsener Hybridkulturen werden ständig auf die Bildung von Antikörpern hin untersucht.

Positive Hybridzellkulturen werden dann mit Hilfe bekannter Verfahren, vorzugsweise aber mit Hilfe des limitierenden Verdünnungsverfahrens vereinzelt und anschliessend in geeigneten Kultivierungsmedien kloniert.

Die Ueberstände der gewachsenen Zellklone werden ebenfalls auf die Bildung von Antikörpern hin überprüft.

Das Screening der gemäss der vorangegangenen Beschreibung hergestellten erfindungsgemässen Hybridoma-Zellklone auf die Bildung geeigneter monoklonaler Antikörper erfolgt vorzugsweise mit Hilfe eines der üblicherweise für diesen Zweck verwendeten Immunassays, wie z.B. einem Enzym-gekoppelten Immunassay oder einem Radioimmunassay.

Beim Enzym-gekoppelten Immunassay werden die zuvor näher charakterisierten Hapten-Konjugate zunächst an einen festen Träger adsorbiert. Die verbleibenden freien Bindungsstellen werden anschliessend durch Zugabe von Carriermolekülen abgesättigt und damit blockiert.

Zum Nachweis monoklonaler Antikörper werden Aliquots der Ueberstände besagter Hybridoma-Zellklone mit den Träger-gebundenen Haptenkonjugaten inkubiert.

Ein weiterer Gegenstand der vorliegenden Erfindung betrifft die Herstellung monoklonaler Antikörper unter Verwendung an sich bekannter Verfahren, die dadurch gekennzeichnet sind, dass man die zuvor näher charakterisierten erfindungsgemässen Hybridomazellinien oder aber Klone oder Subklone davon, welche die erfindungsgemässen Antikörper synthetisieren und in das umgebende Medium sezernieren, in vitro oder in vivo mit Hilfe bekannter Verfahren kultiviert.

Die in-vitro-Kultivierung der erfindungsgemässen Hybridoma-Zellen erfolgt in geeigneten Kultivierungsmedien, insbesondere in den üblicherweise verwendeten, standardisierten Kulturmedien wie z.B. Dulbecco's Modified Eagle Medium (DMEM) oder RPMI 1640 Medium, die gegebenenfalls durch Zugabe von Säuger-Seren, wie z.B. Foetales Kälberserum, oder durch wachstumsfördernde Zusätze und Spurenelemente ergänzt werden können.

Die Isolierung der monoklonalen Antikörper beginnt vorzugsweise mit einer Präzipitation der Immunglobulinfraktion aus den jeweiligen Ueberständen der Hybridomakulturen, z.B. mit Hilfe von Ammoniumsulfat. Es schliessen sich weitere Aufarbeitungs- und Reinigungsschritte an, die dem Fachmann auf diesem Gebiet bekannt sind und die beispielsweise die Verwendung chromatographischer Methoden, wie z.B. Gelfiltration, Ionenaustausch-Chromatographie, DEAE-Cellulose Chromatographie, Protein A oder Immunaffinitätschromatographie miteinschliessen.

Grosse Mengen der erfindungsgemässen monoklonalen Antikörper können aber auch mit Hilfe von in vivo Verfahren gewonnen werden.

So ist es beispielsweise möglich Antikörper-produzierende Hybridoma-Zellklone in geeignete Säugetiere zu injizieren, welche die Entwicklung von Antikörper-produzierenden Tumoren in den behandelten Tieren induzieren. Nach einen Zeitraum von 1 bis 3 Wochen können die Antikörper aus den Körperflüssigkeiten der so behandelten Tiere isoliert werden.

In einer besonderen Ausführungsform der vorliegenden Erfindung wird weiblichen Balb/c Mäusen, die gegebenenfalls mit einem Kohlenwasserstoff wie z.B. Pristan vorbehandelt wurden, ein erfindungsgemässer Hybridoma-Zellklon intraperitoneal injiziert.

Ein bis drei Wochen nach der Injektion des Hybridoma-Zellklons wird die Ascitesflüssigkeit gesammelt und bis zur weiteren Aufbereitung aufbewahrt.

Die Isolierung der monoklonalen Antikörper erfolgt in genau analoger Weise zu der zuvor beschriebenen Isolierung aus den Ueberständen in vitro kultivierter Hybridomas.

Ein weiterer Gegenstand der vorliegenden Erfindung betrifft die Verwendung der erfindungsgemässen Antikörper in einem der gebräuchlichen Immunassays zum Nachweis von Atrazin und/oder Atrazinderivaten bzw. von Hydroxyatrazin und/oder Hydroxyanalogen von Atrazinderivaten sowie zur Differenzierung von Atrazin und/oder Atrazinderivaten gegenüber ihren inaktiven Abbauprodukten in Boden-, Luft- und Wasserproben sowie gegebenenfalls in Extrakten aus Pflanzen oder anderem biologischem Material.

Die erfindungsgemässen monoklonalen Antikörper können somit in allen bekannten Immunassays verwendet werden, die auf der spezifischen Bindung zwischen Antigen und dem entsprechenden monoklonalen Antikörper aufbauen, wie z.B. in einem Radioimmunassay (RIA), einem Enzym-gekoppelten Immunassay (ELISA), einem Immunfluoreszenz-Test etc.

Beim RIA Test kann der erfindungsgemässe monoklonale Antikörper als solcher oder aber in Form eines radioaktiv markierten Derivates verwendet werden. Dabei können alle bis heute bekannten Modifikationen des RIA Tests für den Nachweis der im Rahmen dieser Erfindung relevanten Zielsubstanzen verwendet werden, wie z.B. ein RIA Test in homogener oder fester Phase, ein heterogener RIA Test sowie ein einfacher oder doppelter ('Sandwich') RIA Test mit direktem oder indirektem (kompetitiven) Nachweis des Antigens. Das gleiche gilt auch für die Verwendung eines enzymgekoppelten Immunassays.

Bevorzugt im Rahmen dieser Erfindung ist die Verwendung eines erfindungsgemässen monoklonalen Antikörpers in einem kompetitiven Immunassay zum Nachweis von Atrazin und/oder Atrazinderivaten bzw. ihrer Hydroxy-Analogen.

Das Prinzip des kompetitiven Immunassays beruht auf einer Konkurrenz zwischen einem markierten bzw. einem an einen festen Träger gebundenen Antigen und einem freien Antigen um die relevanten Bindungsstellen auf dem Antikörpermolekül.

Grundsätzlich unterscheidet man zwei Möglichkeiten zur Durchführung dieses kompetitiven Immunassays.

a) Das erste Verfahren beruht auf der Konkurrenz zwischen dem an einen festen Träger gebundenen Antigen und freiem Antigen um die freien Bindungsstellen auf dem Antikörper, der mit einem Marker versehen ist. Die Bindung des Antigens an einen festen Träger kann dabei entweder direkt oder aber über ein Carriermolekül erfolgen.

Die Bestimmung der Konzentration an freiem Antigen erfolgt in diesem Fall über die Abnahme des markierten, an das Träger-fixierte Antigen gebundenen Antikörpers.

Diese Abnahme ist proportional der in der Probe enthaltenen Menge an freiem Antigen.

b) Ein alternatives Verfahren basiert darauf, dass freies und markiertes Antigen miteinander um die relevanten Bindungsstellen des Antikörpers konkurrieren, der in diesem Fall an einen festen Träger gebunden vorliegt.

Die Bestimmung der Konzentration an freiem Antigen erfolgt über die Abnahme an markiertem Antigen, die in Abhängigkeit von der Konzentration an freiem Antigen variiert.

Als festes Trägermaterial, das für die Bindung des Antigens oder des Antikörpers geeignet ist, kommen beispielsweise die Plastikoberfläche einer Mikrotiterplatte oder eines Teströhrchens, die Oberfläche von Kugeln aus Polystyren, Polypropylen, Polyvinylchlorid, Glas oder Kunststoff oder aber die Oberfläche von Filterpapier-, Dextran-Cellulose- oder Nitrozellulose-Streifen oder ähnliche Materialien in Betracht. Diese werden mit einem der erfindungsgemässen monoklonalen Antikörper oder einem Antigen überzogen, wobei die Bindung an das Trägermaterial durch einfache Adsorption oder aber gegebenenfalls nach vorangegangener Aktivierung des Trägermaterials mit z.B. Glutaraldehyd oder Cyanogenbromid vermittelt werden kann.

Besonders bevorzugt im Rahmen dieser Erfindung ist die Verwendung des erfindungsgemässen monoklonalen Antikörpers in einem Enzym-gekoppelten Immunassay [ELISA ('Enzyme Linked Immuno Sorbent Assay)].

Dabei kann der erfindungsgemässe monoklonale Antikörper als solcher oder in Form eines Enzym-gekoppelten Derivates verwendet werden.

Der ELISA Assay basiert entweder auf der Verwendung eines Enzym-gekoppelten Derivates des erfindungsgemässen Antikörpers oder aber von an sich bekannten Enzym-gekoppelten Antikörpern, die ein Epitop eines erfindungsgemässen Antikörpers erkennen und daran binden.

Besonders bevorzugt im Rahmen dieser Erfindung ist die Verwendung eines ELISA-Assays, bei dem eines der zuvor beschriebenen Trägermaterialien zunächst mit einem Antigen beschichtet wird. Das trägergebundene Antigen wird anschliessend mit einer Testlösung inkubiert, die das nachzuweisende Antigen sowie einen der erfindungsgemässen Antikörper enthält. Das nachzuweisende Antigen kann dabei entweder in freier Form oder aber als Bestandteil einer Wasser- oder Bodenprobe vorliegen.

Nach einer Inkubationszeit von 10 Minuten bis 2 Stunden wird der gesamte Ansatz mit einem enzymmarkierten Antikörper inkubiert, der den erfindungsgemässen monoklonalen Antikörper erkennt und an diesen bindet. Ein Beispiel eines solchen enzymmarkierten Antikörpers ist ein Phosphatase-markiertes Ziegen anti-Schaf Immunglobulin, oder ein entsprechender Ziegen anti-Maus Antikörper, die kommerziell bezogen werden können.

Die Menge des gebundenen Antikörperproteins lässt sich anhand einer Enzym-Substrat-Reaktion, z.B. mit Hilfe spektroskopischer Verfahren bestimmen.

Ebenfalls bevorzugt im Rahmen dieser Erfindung ist ein ELISA-Test, der auf der Konkurrenz von markiertem sowie von freiem Antigen um den an eines der zuvor genannten Trägermaterialien gebundenen Antikörper beruht.

Der Anteil an freiem Antigen, der in einer bestimmten Probe vorliegt, wird in diesem Fall über die Abnahme an markiertem Antigen bestimmt, die umso präziser ist, je mehr freies Antigen die Probe enthält.

Eine weitere Möglichkeit zur Durchführung des ELISA-Assays umfasst die folgenden Verfahrensmassnahmen:

Einer der zuvor beschriebenen Carrier, der mit einem der erfindungsgemässen Antikörper beschichtet ist, wird mit einer Testlösung inkubiert, die das nachzuweisende Antigen enthält. Danach wird der gesamte Ansatz mit einem polyklonalen Immunserum gegen besagtes Antigen, beispielsweise einem Immunserum vom Schaf, inkubiert und die gebundenen Antikörper des polyklonalen Serums werden mit Hilfe Enzym-markierter Antikörper, die diese erkennen und binden, entwickelt. Die Menge des gebundenen Proteins wird anhand einer Enzym-Substrat Reaktion bestimmt.

Ein Beispiel eines solchen Enzym-markierten Antikörpers ist ein Phosphatase-markiertes Ziegen anti-Schaf Immunglobulin, das kommerziell erhältlich ist.

Eine weitere Möglichkeit zur Durchführung des ELISA-Assays besteht in der Inkubation eines der zuvor beschriebenen Trägermaterialien, das mit einem der erfindungsgemässen monoklonalen Antikörper beschichtet ist, mit einer Testlösung sowie einer zweiten Lösung, die einen Enzym-konjugierten monoklonalen Antikörper enthält.

Der freie monoklonale Antikörper erkennt dabei ein anderes Epitop auf dem Antigen als der Enzym-gekoppelte Antikörper. Die Menge des gebundenen Enzyms kann mit Hilfe einer Enzym-Substrat Reaktion bestimmt werden, z.B. in Form einer Farbänderung, die optisch wahrgenommen werden kann.

Die Farbänderung ist dabei proportional der Menge des Antigens in der Testlösung.

Darüberhinaus kann der erfindungsgemässe Antikörper auch in einem ELISA Test verwendet werden, bei dem dieser mit einem Enzym markiert ist und der Carrier mit einem monoklonalen anti-Antigen Antikörper beschichtet ist, der ein anderes Epitop erkennt als der erfindungsgemässe monoklonale Antikörper.

Die vorliegende Erfindung betrifft weiterhin Mittel für die qualitative und quantitative Bestimmung von Atrazin und/oder Atrazinderivaten bzw. von Hydroxyanalogen von Atrazin und/oder Atrazinderivaten, wie z.B. Hydroxyatrazin, Hydroxypropazin, Hydroxysimazin etc., in Form eines Test-Kits, die neben den erfindungsgemässen monoklonalen Antikörpern und/oder deren Derivaten, gegebenfalls auch noch andere monoklonale oder polyklonale Antikörper, insbesondere aber markierte monoklonale oder polyklonale Antikörper, sowie weitere Zusätze enthalten können.

Besonders bevorzugt im Rahmen dieser Erfindung sind Test-Kits, die auf einem der üblicherweise verwendeten Immunassays basieren, ausgewählt aus der Gruppe bestehend aus Radioimmunassay, Enzym-gekoppelter Immunassay und Chemilumineszenzassay.

Ganz besonders bevorzugt sind Test-Kits, bei denen der Nachweis von Atrazin und/oder Atrazinderivaten bzw. von Hydroxyanalogen von Atrazin und/oder Atrazinderivaten, wie z.B. Hydroxyatrazin, Hydroxypropazin, Hydroxysimazin etc., auf einem kompetitiven Immunassay, insbesondere aber auf einem Enzym-gekoppelten Immunassay (ELISA) beruht.

Test-Kits für einen radioimmunologischen Nachweis von Atrazin und/oder Atrazinderivaten bzw. von Hydroxyanalogen von Atrazin und/oder Atrazinderivaten, wie z.B. Hydroxyatrazin, Hydroxypropazin, Hydroxysimazin etc., können beispielsweise die folgenden Bestandteile enthalten:

(a) ein geeignetes Trägermaterial, das unbeschichtet oder aber mit einem der erfindungsgemässen Antikörper oder einem Antigenkonjugat beschichtet sein kann;

(b) gegebenenfalls gefriergetrocknete oder konzentrierte Lösungen eines der erfindungsgemässen Antikörper und/oder eines radioaktiv markierten Derivates davon oder radioaktiv markiertes Antigen oder standardisierte Lösungen des Antigens;

(c) Pufferlösungen und

(d) gegebenenfalls Polypeptide, Detergentien und weitere Zusätze, die beispielsweise eine unspezifische Adsorption und Aggregatbildung verhindern sowie

(e) Pipetten, Reaktionsgefässe, Eichkurven, Beipackzettel etc.

Test-Kits für den immunologischen Nachweis von Atrazin und/oder Atrazinderivaten bzw. von Hydroxyanalogen von Atrazin und/oder Atrazinderivaten, wie z.B. Hydroxyatrazin, Hydroxypropazin, Hydroxysimazin etc., die auf einem Enzym-gekoppelten Immunassy (ELISA) basieren, können beispielsweise die folgenden Bestandteile enthalten:

(a) ein geeignetes Trägermaterial, das unbeschichtet oder aber mit einem der erfindungsgemässen Antikörper oder einem Antigenkonjugat beschichtet sein kann;

(b) gegebenenfalls gefriergetrocknete oder konzentrierte Lösungen eines der erfindungsgemässen Antikörper und/oder eines zweiten Enzym-markierten monoklonalen oder polyklonalen Antikörpers, der gegen das zu bestimmende Antigen oder einen das Antigen erkennenden Antikörper gerichtet ist;

(c) Enzymsubstrate in fester oder gelöster Form;

(e) das Antigen oder standardisierte Lösungen des Antigens;

(f) Pufferlösungen;

(g) gegebenenfalls Polypeptide, Detergentien und weitere Zusätze, die beispielsweise eine unspezifische Adsorption und Aggregatbildung verhindern sowie

(h) Pipetten, Reaktionsgefässe, Eichkurven, Farbtafeln, Beipackzettel, etc.

Ein Test-Kit für den Nachweis von Atrazin und/oder Atrazinderivaten bzw. von Hydroxyanalogen von Atrazin und/oder Atrazinderivaten, wie z.B. Hydroxyatrazin, Hydroxypropazin, Hydroxysimazin etc., der auf einem Chemilumineszenztest basiert, kann beispielsweise die folgenden Bestandteile enthalten:

(a) ein geeignetes Trägermaterial, das unbeschichtet oder aber mit einem der erfindungsgemässen Antikörper oder einem Antigenkonjugat beschichtet sein kann;

(b) gegebenenfalls gefriergetrocknete oder konzentrierte Lösungen eines der erfindungsgemässen Antikörper und eines zweiten polyklonalen Antikörpers, der in der Lage ist, den erfindungsgemässen ersten Antikörper zu erkennen und der mit einem chemilumineszierenden Marker verknüpft ist;

(c) Lösungen enthaltend eine Komponente, die die Emission von Licht auslöst, wie z.B. $H_2O_2$ und NaOH;

(d) Pufferlösungen;

(e) gegebenenfalls Polypeptide, Detergentien und weitere Zusätze, die eine unspezifische Adsorption und Aggregatbildung verhindern sowie

(f) Pipetten, Reaktionsgefässe, Beipackzettel, etc.

Trägermaterialien, die im Rahmen der vorliegenden Erfindung Verwendung finden können, umfassen in erster Linie unlösliche, polymere Materialien, ausgewählt aus der Gruppe bestehend aus Polystyren, Polyethylen, Polypropylen, Polyester, Polyacrylnitril, Polyvinylchlorid, Polyacrylamid, Nitrocellulose, quervernetztes Dextran, fluorierte Harze, Agarose, quervernetzte Agarose, Polysaccharide, etc. Daneben sind aber auch andere Materialien denkbar, wie z.B. Glas, Metall, Netzgewebe auf Nylonbasis, etc.

Die zuvor im einzelnen genannten Trägermaterialien können sehr unterschiedlich ausgestaltet sein und in Abhängigkeit vom jeweils angestrebten spezifischen Verwendungszweck sehr verschiedenartige Formen aufweisen. Diese umfassen beispielsweise Schalen, Kugeln, Platten, Stäbchen, Zellen, Fläschchen, Röhrchen, Fasern, Netze, etc.

Häufige Verwendung bei der Herstellung von Test-Kits finden beispielsweise Mikrotiterplatten aus durchsichtigen Plastikmaterialien, wie z.B. Polyvinylchorid oder Polystyren, die unbeschichtet oder aber mit einem der erfindungsgemässen Antikörper, mit freiem Antigen oder einem Antigenkonjugat beschichtet sein können. Ebenso verwendet werden Kügelchen, Röhrchen oder Stäbchen aus Polystyren sowie Polystyrenlatex, wobei das umgebende Latexmaterial via Zentrifugation von den Polystyrenpartikeln abgetrennt werden kann.

Einen weiteren Bestandteil des erfindungsgemässen Testkits bilden Marker oder Indikatoren, mit deren Hilfe es möglich ist, das Vorliegen einer Komplexbildungsreaktion, insbesondere aber einer Immunreaktion nachzuweisen, die vorzugsweise zu einem Antigen-Antikörper-Komplex oder aber zu einem Ligand-Rezeptor-Komplex führt, wobei neben qualitativen gegebenenfalls auch quantitative Aussagen über das nachzuweisende Antigen möglich sind. Als Marker oder Indikatoren kommen sowohl einzelne Atome als auch Moleküle in Betracht, die entweder direkt oder aber indirekt an der Erzeugung eines nachweisbaren Signals beteiligt sein können. Diese Marker oder Indikatoren können entweder direkt mit dem nachzuweisenden Antigen oder mit einem der erfindungsgemässen monoklonalen Antikörper verknüpft sein oder aber in diese eingebaut vorliegen. Sie können aber auch als Einzelsubstanzen oder als Bestandteil einer separaten Verbindung vorliegen, die weder das nachzuweisende Antigen selbst noch einer der erfindungsgemässen monoklonalen Antikörper ist, die ihrerseits aber in der Lage ist mit dem Rezeptormolekül zu reagieren, z.B. in Form einer Komplexbildung.

Bei diesen separat vorliegenden Verbindungen handelt es sich vorzugsweise um ein zweites Antikörpermolekül, das sowohl monoklonalen als auch polyklonalen Ursprungs sein kann, um ein Komplementprotein oder Fragmente davon, um S. aureus protein A, etc. Diese separaten Verbindungen erkennen und binden spezifisch an ein Rezeptormolekül, wie z.B. das nachzuweisende Antigen oder einen der erfindungsgemässen monoklonalen Antikörper, vorzugsweise aber an ein Rezeptormolekül, das in Form eines Komplexes vorliegt.

In vielen Fällen sind weitere, zusätzliche Reagentien notwendig, die dann erst im Zusammenwirken mit dem Marker zu einem nachweisbaren Signal führen. Dies gilt insbesondere dann, wenn Enzyme involviert sind.

Marker oder Indikatoren, die im Rahmen der vorliegenden Erfindung verwendet werden können, sind dem Fachmann auf dem Gebiet der Immunologie und Immunchemie bestens bekannt. Sie umfassen beispielsweise radioaktiv markierte Elemente oder Substanzen, Enzyme oder chemilumineszierende Substanzen. Die folgende Aufzählung möglicher Marker oder Indikatoren soll lediglich dazu diesen, die grosse Vielfalt der verwendbaren Substanzen und Reagentien beispielhaft zu veranschaulichen, ohne dadurch jedoch den Erfindungsgegenstand in irgendeiner Weise einzuschränken.

Geeignete Marker oder Indikatoren sind beispielsweise innerhalb der Gruppe der radioaktiven Elemente zu finden. Dabei sind insbesondere solche Elemente bevorzugt, die entweder selbst $\gamma$ Strahlen emittieren, wie z.B. $^{124}J$, $^{125}J$, $^{128}J$, $^{132}J$, $^{51}Cr$ oder aber eine Emittierung dieser Strahlen induzieren, wie z.B. $^{11}C$, $^{18}F$, $^{13}N$. Ebenfalls geeignet sind sog. β-Stahler wie $^{111}In$, $^{14}C$ und $^{3}H$.

Weitere geeignete Marker umfassen chemilumineszierende Substanzen, insbesondere aber fluoreszierende Substanzen, die sehr einfach auf chemischem Wege mit dem Antigen oder einem Antikörper verbunden werden können ohne diesen zu denaturieren. Das entstehende Fluorochrom lässt sich sehr einfach mit Hilfe fluorometrischer Verfahren nachweisen. Im einzelnen zu nennen sind an dieser Stelle Fluorochrome ausgewählt aus der Gruppe bestehend aus Fluoresceinisocyanat, Fluoresceinisothiocyanat, 5-Dimethylamino-1-naphthalinsulfonylchlorid, Tetramethylrhodaminisothiocyanat, Lissamin, Rhodamin 8200 Sulfonylchlorid, etc.

Weitere fluoreszierende Agentien sowie eine Beschreibung von Analysetechniken findet sich bei DeLuca, "Immunofluorescence Analysis", in: Antibody As a Tool, Marchalonis et al, John Wiley & Sons, Ltd., pp 189-231 (1982).

Besonders bevorzugt im Rahmen dieser Erfindung ist die Verwendung von Enzymen als Marker- oder Indikatorsubstanzen, wie z.B. Meerrettichperoxidase, alkalische Phosphatase, β-D-Galaktosidase, Glucoseoxidase, Glucoamylase, Carbonsäureanhydrase, Acetylcholinesterase, Lysozym, Malatdehydrogenase, Glucose-6-phosphatdehydrogenase, etc. Bei der Verwendung von Enzymen als Markersubstanzen ist es notwendig zusätzliche Reagentien zuzugeben, die es erlauben, die Bildung eines Immunkomplexes über die Enzymaktivität zu verfolgen sowie gegebenenfalls ein Stop-Reagenz, mit welchem die Enzymreaktion beendet werden kann.

Besonders bevorzugt sind dabei Reagentien, die zu einer Farbreaktion führen. Im Falle der Meerrettichperoxidase sei an dieser Stelle beispielhaft Hydrogenperoxid genannt, das in Kombination mit einem zusätzlichen, oxidierten Farbstoffprecursor wie z.B. Diaminobenzidin oder o-Phenylendiamin, zu einer Braun bzw. Gelbfärbung führt. Bei Verwendung der Glucoseoxidase als Markersubstanz kann beispielsweise 2,2′-Azino-di-(3-ethyl-benzthiazolin-6-sulfonsäure) [ABTS] als Substrat eingesetzt werden.

Ein weiterer Gegenstand der vorliegenden Erfindung betrifft somit die Verwendung von Test-Kits, die zumindest einen der erfindungsgemässen monoklonalen Antikörper als Reagenz enthalten, zum schnellen und effektiven, qualitativen und/oder quantitativen Nachweis von Atrazin und/oder Atrazinderivaten bzw. von Hydroxyanalogen von Atrazin und/oder Atrazinderivaten, wie z.B. Hydroxyatrazin, Hydroxypropazin, Hydroxysimazin etc. sowie zur Differenzierung von Atrazin und/oder Atrazinderivaten gegenüber ihren Abbauprodukten.

I. Nichtlimitierende Ausführungsbeispiele

Beispiel 1: Synthese von Atrazin/Hydroxyatrazinkonjugaten

1.1: Triazinyl-Valeriansäure-Kupplungskomponenten

a) 2-chlor-4-isopropylamino-6-(1-carboxybutyl-4-amino)-s-triazin

Zu einer Mischung von 10,35 g 2,6-Dichlor-4-isopropylamino-s-triazin und 6,15 g 5-Aminovaleriansäure in 150 ml trockenen Chloroforms werden 15,66 ml Diazobicyclo[5.4.0]undec-5-en gegeben, 1 Stunde bei Rückflusstemperatur gerührt und die Reaktionslösung am Vakuum eingedampft (Zimmertemperatur). Der ölige Rückstand wird bei 0°C mit 60 ml 2N Salzsäure verrührt, nach zwei-stündigem Stehen der Niederschlag filtriert, mit Wasser und Diäthyläther gewaschen und getrocknet. Man erhält 4,3 g des gewünschten Produktes, 2-Chlor-4-isopropylamino-6-(1-carboxybutyl-4-amino)-s-triazin (Smp. 191-192°C).

b) 2-Hydroxy-4-isopropylamino-6-(1-carboxybutyl-4-amino)-s-triazin · Hydrochlorid

1 g 2-chlor-4-isopropylamino-6-(1-carboxybutyl-4-amino)-s-triazin wird während 4 Stunden bei Raumtemperatur in 7 ml 6 N Salzsäure gerührt, am Vakuum bei 45°C eingeengt, die Kristalle filtriert und getrocknet. Man erhält 0,98 g des gewünschten Produktes, 2-Hydroxy-4-isopropylamino-6-(1-carboxybutyl-4-amino)-s-triazin · Hydrochlorid (Smp. 149-151°C).

1.2: <u>Triazin-Protein-Konjugate</u>

Die Atrazin- und Hydroxyatrazinderivate werden entweder an "Bovine Serum Albumine" (BSA; Fluka) oder an "Keyhole Limpet Hemocyanin" (KLH; Calbiochem) konjugiert unter Anwendung der aktivierten Ester-Methode (Kulkarni <u>et al.</u>, 1981).

Im einzelnen wird dabei die Carboxylgruppe des Derivats in N,N-Dimethylformamid (DMF) (6 mg/200 µl) bei Raumtemperatur solubilisiert und anschliessend mit einem 4molaren Ueberschuss von $\alpha$-Hydroxysuccinimid (9.1 mg/200 µl) sowie N,N′-Dicyclohexylcarbodiimid (16 mg/200 µl) versetzt.

Das bei der Reaktion gebildete Präzipitat wird durch 3 minütige Zentrifugation mit 12'000 g bei Raumtemperatur entfernt und der aktivierte Ester anschliessend zu BSA oder KLH gegeben, das zuvor in einer Phosphatgepufferten Salzlösung (PBS-Puffer) solubilisiert worden ist. Das molare Verhältnis von [Derivat]/[BSA] beträgt dabei ca. 55/1 (24 mg Derivat/5.4 ml BSA).

Nach 4stündiger Inkubation bei einer Temperatur von 4°C wird das gebildete Präzipitat durch 10 minütige Zentrifugation mit 2'000 g bei 4°C entfernt und der verbleibende Ueberstand ausgiebig gegen PBS dialysiert, bevor er dann für die Immunisierungs-Experimente verwendet wird.

Das Ausmass der Kupplungsreaktion wird mit Hilfe einer SDS-Gelelektrophorese sowie durch Absorptionsspektrophotometrie bestimmt. Das molare Verhältnis von Hydroxyatrazin bzw. Atrazin zu BSA liegt bei ca. 11:1.

Beispiel 2: <u>Immunisierung</u>

Gruppen von jeweils 5 weiblichen BALB/c Mäusen (Tierfarm Sisseln, Schweiz), die zwischen 4 und 6 Wochen alt sind, erhalten 3 Serien von intraperitonealen bzw. subkutanen Injektionen mit KLH-konjugiertem Atrazin oder Hydroxyatrazin (50 µg/Injektion).

Die erste Injektion beinhaltet 0,1 ml des Konjugates in PBS, das in einem Verhältnis von 1:1 mit 0,1 ml komplettem Freund Adjuvanz vermischt ist. 50 µl dieser Injektionslösung werden intraperitoneal injiziert, die restlichen 150 µl subcutan.

Bei der zweiten und dritten Injektionsserie, die 14 bzw. 30 Tage nach der Erstapplikation erfolgt, wird das komplette Freund Adjuvans durch inkomplettes ersetzt.

1 Woche nach der letzten Injektion wird Blutserum von den Versuchstieren entnommen und die Bluttiter mit Hilfe eines ELISA-Tests bestimmt, wobei die Mikrotiterplatten zuvor mit BSA-konjugiertem Hapten beschichtet werden (siehe Abschnitt 6).

Nach einer Ruheperiode von 2 Monaten erfolgt eine weitere einmalige intraperitoneale Injektion des KLH-Konjugates in einer Dosierung von 500 µg/200µl PBS.

Beispiel 3: <u>Fusionsprotokoll</u>

3.1. <u>Gewinnung von "Feeder"-Zellen (peritoneale Makrophagen).</u>

Unbehandelte, ca. 6 bis 8 Wochen alte Balb/c-Mäuse werden einen Tag vor der beabsichtigten Fusion getötet und durch Eintauchen in 70 %igem Alkohol sterilisiert.

Anschliessend wird das Fell und die obere Bauchhaut steril angeschnitten ohne das Peritoneum zu verletzen. Mit einer sterilen 5 ml Plastikspritze und einer sterilen 18-er Injektionsnadel werden 4 ml BSS (ohne $Ca^{2+}$ und $Mg^{2+}$) und 1 ml Luft in die Bauchhöhle injiziert.

Nach leichtem Massieren des Bauches (wobei Spritze und Nadel in der Bauchhöhle verbleiben) wird der zuvor injizierte BSS-Puffer wieder aus dem Peritoneum abgezogen und in ein steriles Falconröhrchen gegeben. Dieses Procedere wird noch 2 mal wiederholt. Die so gewonnenen Makrophagen werden mit Eis gekühlt und anschliessend 2 x mit je 20 ml BSS gewaschen.

Die Makrophagen werden dabei je 10 Min. bei 300 g und einer Temperatur von 5°C zentrifugiert. Das Pellet wird dann in 50 ml HAT-Medium resuspendiert und die Zellsuspension auf 4 Costarplatten mit insgesamt 24 Vertiefungen verteilt (0,5 ml/Vertiefung).

Anschliessend werden die so vorbereiteten Makrophagen bei einer Temperatur von 37°C und einer $CO_2$-Konzentration von 6 % in einem Inkubator aufbewahrt.

Pro Fusionsvorgang werden ca. 4 x $10^6$ Makrophagen benötigt.

### 3.2. Anzucht der Myeloma-Zellinie Sp 2/0-Ag 14

Bei der genannten Myeloma-Zellinie Sp 2/0-Ag 14 handelt es sich um eine Myeloma-Zellinie, die selbst keine Antikörper sezerniert und die bei M. Shulman et al. (1978) beschrieben ist. Diese Myeloma-Zellinie kann bei der "American Type Culture Collection" in Rockville, Maryland bezogen werden.

Pro Fusion benötigt man 50 ml einer gut gewachsenen Kultur, die mindestens 10 Millionen Zellen enthält. Die Kultivierung der Myelomzellen erfolgt vorzugsweise in T 175 "Falcon"-Flaschen (Fa. Beckton & Dickenson).

Einen Tag vor der Fusion wird das Kultivierungsmedium (RPMI 1640) durch frisches RPMI 1640-Medium ersetzt. Am Tag der Fusion werden die Sp 2/0-Ag 14 Zellen geerntet, in ein steriles 50 ml Plastikröhrchen gegeben und 10 Min. bei 300 g und einer Temperatur von 5°C zentrifugiert (MSE Zentrifuge, Modell Chilspin, UK).

Nach der Zentrifugation wird der Ueberstand abgezogen und verworfen. Die Zellen werden 2 x mit je ca. 30 ml BSS-Puffer ($Ca^{2+}$ und $Mg^{2+}$ frei) gewaschen (10 Min. bei 300 g, 5°C) und anschliessend in 5 ml BSS resuspendiert.

Ein Aliquot der Zellsuspension wird zur Bestimmung der Zellzahl entnommen und mit Fluoresceindiacetat (FDA) gefärbt. Bis zur Weiterverwendung werden die Myelomzellen auf Eis aufbewahrt.

### 3.3. Herstellung einer Milzzellsuspension

Die Entnahme der Milz aus einer zuvor gemäss Beispiel 2 immunisierten Balb/c-Maus erfolgt unter sterilen Bedingungen und unter Kühlung mit Eis.

Die zuvor immunisierte Balb/c-Maus wird durch Genickbruch getötet und die Milz steril entnommen. Dazu wird die Maus kurz in 70 %igen Ethanol eingetaucht und mit sterilem Besteck präpariert. Die Milz wird vorsichtig entnommen und auf ein feines Nylonnetz gelegt. Dort wird sie mit einer Schere fein zerschnitten und dann mit Hilfe eines 5 ml Spritzenstempels vorsichtig durch das Netz gedrückt, ohne dabei zu viele Zellen zu zerstören. Während des ganzen Vorgangs wird das Netz mit BSS gespült.

Die so gewonnene Zellsuspension wird in 50 ml Plastikröhrchen gegeben und 10 Min. bei 300 g und einer Temperatur von 5°C zentrifugiert (MSE Zentrifuge, Modell Chilspin; UK). Die Zellen werden anschliessend 2 x mit je 20 ml BSS gewaschen (10 Min.; 300 g; 5°C; MES Chilspin) und das Zellpellet wird nach der Zentrifugation in 10 ml BSS resuspendiert.

Bis zur Fusion mit Sp 2/0-Ag14 Myelomzellen werden die Milzzellen auf Eis belassen.

### 3.4. Fusion: Milzzellen und Sp 2/0-Ag 14 Myelomzellen

Das Verhältnis von Myelomzellen zu Milzzellen sollte für die Fusion 1:10 betragen.

Milzzellen (in BSS-Puffer) und Sp 2/0-Ag14 Myelomzellen (in BSS-Puffer) werden in dem angegebenen Verhältnis zusammengegeben und 10 Min. bei 300 g und einer Temperatur von 5°C zentrifugiert (MSE Zentrifuge, Modell Chilspin). Das Pellet wird nochmals in BSS-Puffer resuspendiert und die Suspension anschliessend erneut zentrifugiert.

Das Pellet wird vorsichtig aufgerührt und in 37°C warmes Wasserbad gestellt. Es wird dann 1 ml vorgewärmtes und steriles PEG-4000 (MERCK) über einen Zeitraum von 60 Sek. tropfenweise zu den Zellen zugegeben, wobei der gesamte Ansatz ständig bewegt wird. Die Zellen werden anschliessend noch 30 Sek. lang weitergeschüttelt, bevor 5 ml einer zuvor erwärmten BSS-Puffers (ohne $Ca^{2+}$, $Mg^{2+}$) ebenfalls tropfenweise über einen Zeitraum von ca. 5 Min. unter ständigem Rühren dazugegeben werden.

Die auf die beschriebene Weise fusionierten Zellen werden dann abzentrifugiert (10 Min.; 300 g; 20°C, MSE Zentrifuge, Modell Chilspin), der Ueberstand wird abgezogen und verworfen. Das Zellpellet wird in 50 ml HAT-Medium resuspendiert und die so erhaltene Zellsuspension auf die vorbereiteten 4 Costarplatten (Mikrotiterplatten mit 24 Vertiefungen, Durchmesser pro Vertiefung 24 mm; Gesamtfläche für Zellwachstum 2,0 $cm^2$) verteilt (0,5 ml/Vertiefung).

Die Inkubation der Costarplatten erfolgt bei einer Temperatur von 37°C und bei einer $CO_2$-Konzentration von 6 %.

Beispiel 4: Kultivierung der Hybridzellen

Am 1. Tag nach der Zellfusion wird 1 ml HAT-Medium pro Vertiefung zu den Kulturplatten zugegeben. 3 bis 4 Tage nach der Zellfusion erfolgt eine mikroskopische Kontrolle der fusionierten Zellen. Gleichzeitig wird das verbrauchte Medium abgesaugt und durch 1 ml frisches HAT-Medium ersetzt. Nach weiteren 3 Tagen (6 - 7 Tage nach der Zellfusion) erfolgt ein erneuter Wechsel des Kulturmediums. Ab dem 7. bis 10 Tag nach der Zellfusion wird jede Vertiefung mikroskopisch nach Hybriden abgesucht und 2 bis 3x wöchentlich das Medium erneuert.

Sobald in einer Vertiefung Hybride gewachsen sind, kann das HAT Medium dort durch HT-Medium ersetzt werden. Der Ueberstand von gewaschenen Hybridkulturen (mindestens 10 % der Vertiefung) wird mit einer sterilen Pasteurpipette abgehoben und auf das Vorhandensein von Antikörpern getestet.

Sobald die Vertiefung mit positiven Hybridkolonien voll bewachsen sind, können diese auf neue Costarplatten in RPMI 1640-Medium transferriert werden, wobei der Inhalt einer vollbewachsenen Vertiefung auf 2 bis 3 neue Vertiefungen verteilt wird.

Beispiel 5: Klonierung der positiven Hybridzellen

Die Zellen einer positiven Vertiefung werden mit Hilfe einer Pipette gelöst und in 1 ml Medium in ein Röhrchen überführt. Anschliessend wird ein Aliquot zur Bestimmung der Zellzahl entnommen und mit FDA angefärbt (Verdünnung 1 : 2 mit FDA: 50 $\mu$l Zellen + 50 $\mu$l Farbstoff). Die bevorzugte Zellzahl liegt bei $10^5$ bis $10^6$ Zellen/ml.

Anschliessend werden die Hybridzellen in einem Verhältnis von 1 : 100 mit HT-Medium verdünnt (z.B. 100 $\mu$l Zellen + 9,9 ml HT-Medium).

In zwei 50 ml Falconröhrchen werden je 25 ml HT-Medium vorgelegt und mit 5 ml einer Macrophagensuspension auf insgesamt 30 ml pro Röhrchen aufgefüllt. Die Macrophagen werden zuvor aus einer Maus isoliert und in 10 ml HT-Medium resuspendiert (vgl. Abschnitt 3.1).

In diesen die Macrophagen enthaltenden Falcon-Röhrchen werden die Hybridzellen verdünnt, bis eine Zelldichte von (i) 270 Zellen/30 ml bzw. (ii) 90 Zellen/30 ml erreicht ist. Anschliessend werden diese Ansätze auf Costarplatten (Mikrotiterplatten mit 96 Vertiefungen) verteilt, wobei je 200 $\mu$l pro Vertiefung zugegeben werden. Dies entspricht einer Zellzahl von (i) 1,8 Zellen/Vertiefung bzw. (ii) 0,6 Zellen/Vertiefung. Pro Verdünnung benötigt man auf diese Weise 1,5 Mikrotiterplatten.

Nach 7 Tagen werden die einzelnen Vertiefungen mikroskopisch kontrolliert und die Vertiefungen die Zellklone enthalten erfasst. Diejenige Verdünnung, bei der ca. 50 % der Vertiefungen Zellklone enthalten wird für den ELISA-Test verwendet. Dies sollte in der Regel die Verdünnung mit 0,6 Zellen/Vertiefung sein.

Nach ca. 7 - 10 Tagen werden die Ueberstände der positiven Vertiefungen (mit Klonen) in einem ELISA-Test auf die Anwesenheit von monoklonalen Antikörpern getestet und die positiven Klone auf Costarplatten (mit 24 Bohrungen) in RPMI 1640-Medium vermehrt. Aliquots dieser positiven Klone werden in flüssigem Stickstoff aufbewahrt.

Beispiel 6: Hybridoma-Screening (ELISA-Test)

Zunächst werden 100 $\mu$l einer Lösung von BSA-konjugiertem Hapten in Natriumcarbonat-Puffer (50 mM, pH 9.6) in die einzelnen Vertiefungen einer Mikrotiterplatte gegeben und dieser Ansatz bei 4°C in einer feuchten Kammer über Nacht inkubiert. Anschliessend werden die Vertiefungen jeweils 5 x mit einem 0,1 % PBS-Tween Puffer gewaschen.

Zur Blockierung der unbesetzten Bindungsstellen auf der Mikrotiterplatte werden 200 $\mu$l einer PBS-BSA Lösung (1 %) in jede Vertiefung gegeben. Dieser Ansatz wird 1 - 2 Stunden bei Raumtemperatur inkubiert und anschliessend mit einem 0,1 %igen PBS-Tween-Puffer gewaschen.

Man gibt dann zu jeder Vertiefung 200 $\mu$l des Hybridoma-Ueberstands, der im Verhältnis 1:2 mit PBS-Tween (0,1 %) verdünnt ist, hinzu und inkubiert den gesamten Ansatz für 2 Stunden bei Raumtemperatur. Anschliessend werden die Vertiefungen erneut 5 x mit einem 0,1 %igen PBS-Tween-Puffer gewaschen.

Es folgt die Inkubation mit Phosphatase-konjugiertem anti-Maus Antikörper (Kirkegaard & Perry Lab.). Zunächst werden 100 $\mu$l eines über eine Affinitätschromatographie gereinigten Ziegen Antikörpers gegen Mäuse IgG, der 1 : 1500 in PBS-Tween (0,1 %) verdünnt vorliegt (Kirkegaard & Perry Laboratories) und mit alkalischer Phosphatase markiert ist, zu jeder Vertiefung hinzugegeben.

Die Inkubationszeit beträgt 1,5 Stunden bei Raumtemperatur. Anschliessend werden die einzelnen Vertiefungen erneut mit PBS-Tween (0,1 %) gewaschen (5 x).

Danach werden 150 $\mu$l einer substrathaltigen Lösung (1 mg/ml p-Nitrophenylphosphat) in jede Vertiefung zugegeben. Nach einer Inkubationszeit von 2 Stunden im Dunkeln erfolgt die spektroskopische Bestimmung bei 405 nm. Positive Hybridoma-Zellen, die einen spezifischen Antikörper sezernieren geben ein starkes positives Signal bei der gewählten Wellenlänge.

### Beispiel 7: Expansion der Hybridomazellen in der Maus

Für die Anregung der Ascites-Produktion werden weibliche Balb/c Mäuse (20 - 25 mg) (Tierfarm Sisseln, CH) mit 0.3 ml Pristan Oel (Aldrich Chemical) vorbehandelt, das intraperitoneal injiziert wird.

1 bis 3 Wochen nach der Pristan Applikation erhalten die Mäuse eine zweite Injektion (0,2 ml Pristan-Oel, i.p.). Gleichzeitig mit dieser 2. Injektion erhalten die Tiere $2 \times 10^6$ Hybridomazellen in 0,2 ml PBS.

Die aus dieser Behandlung resultierende Ascitesflüssigkeit wird gesammelt, bei 800 g zentrifugiert und bei einer Temperatur von -20°C aufbewahrt. Nach dem Auftauen wird die Ascitesflüssigkeit 1 Stunde bei 30'000 g zentrifugiert. Die oberste Schicht, die vorwiegend Lipide enthält, wird entfernt. Anschliessend wird die Proteinkonzentration bestimmt und auf einen Wert von 10 mg/ml eingestellt durch Zugabe von PBS.

Die Immunglobulin G Fraktion (IgG) wird durch tropfenweise Zugabe von 0,9 Volumenteilen einer gesättigten Ammoniumsulfatlösung bei 0°C präzipitiert. Nach 1 Stunde wird die IgG-Fraktion pelletiert durch einstündige Zentrifugation bei 22'000 g. Das Pellet wird anschliessend in 20 mM-Tris-HCl Puffer, pH 7,9, der 50 mM NaCl enthält, aufgelöst und gegen den gleichen Puffer über Nacht bei 4°C dialysiert.

Die weitere Aufarbeitung der IgG Fraktion geschieht mit Hilfe einer Anionen-Austauschchromatographie an einer DE-52 Diethylaminoethylcellulose (Whatman) Säule. Die Probe wird 1:2 (v/v) mit 20 mM Tris-HCl, pH 7,9 verdünnt, bis eine NaCl Endkonzentration von 25 mM erreicht ist und 10 mg Protein/ml Gel werden auf die Säule aufgetragen. Die Elution wird erreicht durch Erhöhung der NaCl-Konzentration von 25 mM auf 200 mM (linearer Gradient). Im allgemeinen erfolgt die Elution monoklonaler Antikörper im Bereich von 80 mM NaCl.

Die Fraktionen werden über Nacht bei einer Temperatur von 4°C gegen PBS dialysiert und bei -70°C aufbewahrt. Der Reinheitsgrad wird mit Hilfe einer Natriumdodecylsulphat Polyacrylamid-Gelelectrophorese (SDS-PAGE) bestimmt, sowie durch isoelektrische Fokussierung.

Im vorliegenden Fall liegt der Reinheitsgrad bei >90 %.

### Beispiel 8: Atrazin und Hydroxyatrazin-Nachweis

Der Nachweis von Atrazin und Hydroxyatrazin erfolgt mit Hilfe eines zweistufigen, kompetitiven ELISA-Tests.

BSA-konjugiertes Hapten wird zunächst in einem Natriumcarbonatpuffer (pH 9,6) (0,2 μg/Vertiefung) an Mikrotiterplatten adsorbiert und über Nacht bei einer Temperatur von 4°C inkubiert. Die restlichen freien Bindungsstellen des festen Trägermaterials werden dann durch Zugabe von BSA in Form einer 1 % Lösung blockiert.

Die Platten werden anschliessend mit PBS gewaschen, das mit 0,1 % (v/v) Polysorbat 20 angereichert ist (PBS-Tween).

50 μl der zuvor gereinigten monoklonalen Antikörper (2 μg/ml) oder aber des Ueberstandes der Zellklone (in einer Verdünnung von 1:5 bis 1:22,5) werden a) mit 950 μl einer Standardlösung, die einen steigenden Anteil an Triazin bzw. Triazin-Analogen enthält, b) mit triazinhaltigen Wasserproben oder c) triazinhaltigen Bodenextrakten inkubiert. (Alle Verdünnungen werden in PBS-Tween vorgenommen).

Nach einer Inkubationszeit von 1 Stunde bei Raumtemperatur werden 200 μl des Gemisches zu jeder Vertiefung der Mikrotiterplatte zugegeben und der gesamte Ansatz wird für eine weitere Stunde inkubiert.

Die Vertiefungen werden anschliessend 5 x gewaschen und mit 100 μl/Vertiefung Ziegen-anti-Maus IgG Antikörper der konjugiert an alkalische Phosphatase vorliegt (Verdünnung 1:1500) beschickt und für einen Zeitraum von 1,5 Stunden inkubiert.

Nach erneutem Waschen werden 150 μl/Vertiefung des in 1 mg/ml Diethanolaminpuffer (1 mM, pH 9,8) gelösten Substrates p-Nitrophenylphosphat zu den Vertiefungen zugegeben.

Ein Farbumschlag, der proportional ist zur Menge an Antikörper, der mit dem an die Festphase gebundenen Antigen reagiert hat, wird bei einer Wellenlänge von 405 nm aufgezeichnet. Die Verdünnungen der einzelnen Proben werden so gewählt, dass man ohne Zugabe eines Inhibitors (Bo) Absorptionswerte in einem Bereich zwischen 0.3 und 0.5 erhält. Für die Kontrollen (ohne Antikörper und mit nicht nachweisbaren Mengen an Antigen) ergeben sich Werte von $R \leq 0.005$. Alle Proben werden in dreifacher Ausfertigung bestimmt.

Zur Ermittlung der in einer Probe enthaltenen Menge an Atrazin/Hydroxyatrazin wird zunächst eine Eichkurve erstellt (Abb. 1), wobei B/Bo x 100 gegen die Konzentration an Inhibitor aufgetragen wird. (Bo bedeutet Absorptionsfähigkeit gemessen ohne Zugabe eines Triazininhibitors zum Antikörper, und B Absorptionsfähigkeit bei Zugabe verschieden konzentrierter Triazin-Inhibitoren) Der $I_{50}$-Wert gibt diejenige Konzentration des Antigens an, bei der die Antikörperbindung an die Festphase zu 50 % gehemmt wird. Der $I_{50}$-Wert wird mit Hilfe

EP 0 365 818 B1

eines auf die vorliegenden Verhältnisse speziell angepassten ENZFITTER (Leatherbarrow, Elsevier-Biosoft)Kurvenkalkulationsprogramms bestimmt, basierend auf einer 4 Parameter umfassenden logistischen Kurve (Raab GM, 1983). Auch die quantitative Atrazin bzw. Hydroxyatrazinbestimmung in Boden- oder Wasserproben im Rahmen des ELISA wird mit Hilfe des ENZFITTER-Programms durchgeführt, wobei die Anpassung der Kurve auf Standards basiert, die auf jeder Mikrotiterplatte mitlaufen.

Beispiel 8.1: Analyse von Bodenproben

Aliquots (2 g) standardisierter Bodenproben unterschiedlicher Herkunft (siehe Tabelle II) werden in einem Extraktor 4 Stunden mit 20 ml eines Methanol/Wasser [80/20 (v/v)] Gemisches extrahiert. Für den kompetitiven ELISA werden die Bodenextrakte gewöhnlich in einem Verhältnis von 1:40 in PBS-Tween (0.1 %) verdünnt, um einer möglichen Denaturierung der monoklonalen Antikörper durch das Methanol vorzubeugen. Anschliessend werden die verdünnten Bodenproben (950 µl) auf die zuvor beschriebene Weise mit den spezifischen Antikörpern (50 µl) inkubiert.

Für die HPLC Bestimmung von Hydroxyatrazin werden die Methanolextrakte durch Kationenaustauschchromatographie (z.B. über eine Dowex 5OW-X4 Säule, gefolgt von einer Adsorption an ein Amberlite XAD-2 Polystyrendivinylbenzol Austauscherharz) weiter gereinigt. Abschliessend wird eine Gelfiltration, z.B. über eine Bio-Gel P-2 Säule, durchgeführt. Die Proben werden dann auf die HPLC gegeben und Hydroxyatrazin wird bei einer Wellenlänge von 240 nm bestimmt.

Die vergleichende Atrazinbestimmung wird mit Hilfe der Gaschromatographie, unter Verwendung eines thermo-ionischen Detektors, und/oder anschliessender Massenspektroskopie durchgeführt.

Beispiel 8.2: Analyse von Wasserproben

Für den kompetitiven ELISA werden 100 µl eines 10fach konzentrierten PBS-Tween Puffers zu 850 µl einer Wasserprobe zugegeben. Dieser Ansatz wird schliesslich mit 50 µl des anti-Atrazin Antikörpers MAb 4063-21-1 inkubiert.

Für die HPLC Bestimmung werden die in der Wasserprobe (20 ml) enthaltenen s-Triazine zunächst auf einer RP-18 Vorlaufsäule konzentriert und anschliessend auf einer analytischen RP-18 Säule weiter aufgetrennt. Die Atrazinbestimmung wird bei 230 nm durchgeführt.

II. Ergebnisse

a) Herstellung monokonaler Antikörper

Bereits wenige Tage nach der erfolgten Zellfusion zwischen Myelomazellen und den Milzzellen einer Maus, die zuvor mit dem in Abschnitt 1 beschriebenen Hydroxykonjugat immunisiert wurde, lassen sich in 93 der insgesamt 96 Vertiefungen der Mikrotiterplatten proliferierende Zellkolonien nachweisen. Dies entspricht einer Fusionseffizienz von 97 %.

10 dieser Zellkolonien produzieren monoklonale Antikörper, welche im ELISA-Test eine sehr starke Reaktion zeigen. Diese werden mit Hilfe der limitierten Verdünnungsmethode (vgl. Abschnitt 5) kloniert.

9 der synthetisierten monoklonalen Antikörper gehören in die IgG1 Subklasse, einer in die IgG2b Subklasse.

Die auf die zuvor beschriebene Weise gewonnen monoklonalen Antikörper können aufgrund ihrer Kreuzreaktivität, die in einem ELISA-Test bestimmt wird, in 2 Gruppen unterteilt werden.

In der ersten Gruppe (vertreten durch MAb 4009-85-3) ist die Kreuzreaktivität in der Hauptsache auf Hydroxypropazin beschränkt, wohingegen in der zweiten Gruppe (vertreten durch MAb 4009-77-20) Kreuzreaktivitäten auch mit anderen s-Triazinen auftreten, wie z.B. Hydroxysimazin, Hydroxydesmetryn u.a. (vgl. Tabelle 1).

In beiden Gruppen tritt dagegen keine Kreuzreaktivität mit aktiven Triazinen, wie z.B. Atrazin, auf. Somit bleibt die Bindung der monoklonalen Antikörper auf Verbindungen beschränkt, die eine Hydroxy-Gruppe in Position 2 des Triazinringes aufweisen.

Die untere Nachweisgrenze für Hydroxyatrazin liegt im Bereich von 0,1 ng/ml Puffer für MAb 4009-85-3 bzw. von 0.05 ng/ml Puffer für MAb 4009-77-20. Die entsprechenden $I_{50}$-Werte betragen 0.95 ng/ml und 0.5 ng/ml.

Bei Verwendung von Atrazin-Konjugaten (vertreten durch MAb 4063-21-1) erhält man insgesamt 5 monoklonale Antikörper, die alle ein etwa vergleichbares Ausmass an Kreuzreaktivität zeigen. In diesem Fall findet man eine Kreuzreaktivität mit verschiedenen aktiven s-Triazinen.

Die auftretenden Kreuzreaktivitäten mit hydroxylierten Metaboliten sind dagegen vernachlässigbar gering. (vgl. Tabelle 1)

Für MAb 4063-21-1 liegt die Nachweisgrenze für Atrazin bei 0.05 ng/ml Puffer bei einem $I_{50}$-Wert (50 % Hemmung) von 0.45 ng/ml.

b) Rückgewinnungsstudien

Diese Rückgewinnungsstudien werden durchgeführt, indem man eine bekannte Menge an Hydroxyatrazin oder Atrazin zu einem Methanol/Wasser Bodenextrakt zugibt. Sie dienen in erster Linie dazu, die eventuell störenden Einflüsse von organischem Material während des Immunassays zu untersuchen.

Tab. II zeigt, dass eine fast komplette Rückgewinnung erfolgt, unabhängig von der Zusammensetzung der verwendeten Bodenproben. Sogar bei einem Humusanteil von annähernd 10 % beträgt die Rückgewinnungsrate 97%.

c) Atrazinnachweis in Wasserproben

21 verschiedene Wasserproben wurden mit Hilfe von HPLC und ELISA unter Verwendung von MAb 4063-21-1 analysiert.

Für die HPLC-Bestimmung muss ein Aufkonzentrierungsschritt durchgeführt werden, der bei Verwendung der erfindungsgemässen monoklonalen Antikörper in einem ELISA-Assay entfällt.

Tab. III zeigt, dass die Nachweisgrenze für Atrazin bei Verwendung des Immunassays bei ca. 0.05 ppb liegt. Für die Atrazin-haltigen Proben (> 0,05 ppb) wird die Korrelation zwischen ELISA und HPLC-Bestimmung mittels Regressionsanalyse bestimmt. Dabei wird eine gute Uebereinstimmung zwischen beiden Methoden sichtbar ($r = 0,91$, $p < 0,0005$), wobei die leicht erhöhten Werte bei der ELISA-Bestimmung nicht signifikant sind.

Die Wasserproben wurden darüberhinaus auch auf Spuren von Hydroxyatrazin hin untersucht. Mit keiner der beiden Methoden (HPLC und ELISA) liess sich dieser Metabolit nachweisen.

Hinterlegung:

Die im Rahmen der vorliegenden Erfindung hergestellten und verwendeten Hybridoma-Zellinien wurden bei der als Internationale Hinterlegungsstelle anerkannten 'European Collection of Animal Cell Cultures' (ECACC) in Salisbury, UK, entsprechend den Anforderungen des Budapester Vertrages für die Internationale Anerkennung der Hinterlegung von Mikroorganismen zum Zwecke der Patentierung, hinterlegt. Eine Erklärung zur Lebensfähigkeit der hinterlegten Proben wird durch die besagte Internationale Hinterlegungsstelle ausgefertigt.

| Zellinie | Hinterlegungsdatum | Hinterlegungsnummer* | Datum der Lebensfähigkeitsbescheinigung |
|---|---|---|---|
| Hybridoma Klon 4063-21-1 | 25.08.1988 | 8808/2501 | 25.08.1988 |
| Hybridoma Klon 4009-77-20 | 25.08.1988 | 8808/2502 | 25.08.1988 |
| Hybridoma Klon 4009-85-3 | 25.08.1988 | 8808/2503 | 25.08.1988 |

* Hinterlegungsnummer, ausgegeben durch die oben bezeichnete Internationale Hinterlegungsstelle.

Verwendete Medien und Puffer

A) RPMI 1640-Medium

RPMI 1640 (Seromed) mit folgenden Zusätzen:

| | |
|---|---|
| Kälberserum | 15 % |
| L-Glutamin | 4 mM |
| Gentamycin | 0,01 % |
| Natrium-Pyruvat | 1 mM |
| 2-Mercaptoethanol | 50 $\mu$M |
| Insulin | 5 $\mu$M |
| Transferrin | 5 $\mu$M |
| Selen (ITS) | 5 $\mu$M |

B) HAT-Medium

1 Liter RPMI 1640 Medium mit 20 ml Zusatz von HAT conc. 5o x von Boehringer (Hypoxanthin 680,5 mg/l; Aminopterin 8,8 mg/l; Thymidin 193,8 mg/l)

C) HT-Medium

1 Liter RPMI-1640 Medium mit 20 ml Zusatz von HT conc. 5o x von Boehringer (Hypoxanthin 680,5 mg/l; Thymidin 193,8 mg/l).

D) BSS-Puffer (Earle's Salzlösung, ohne Ca und Mg, pH 7,4)

| | |
|---|---|
| KCl | 7,3 mM |
| NaCl | 116 mM |
| $NaHCO_3$ | 26 mM |
| $NaH_2PO_4 \cdot 2H_2O$ | 1 mM |
| Glucose | 5,5 mM |
| Phenolrot | 48 $\mu$M |

1 % Zusatz (v/v) einer Penicillin/Streptomycin-Lösung (Seromed) (10'000 E Penicillin, 10 mg/ml Streptomycin).

E) Natriumcarbonat-Puffer (pH 9,6)

| | |
|---|---|
| $Na_2CO_3$ | 477 mg |
| $NaHCO_3$ | 879 mg |
| $NaN_3$ (0,5M) | 1,8 ml |
| ad 300 ml $H_2O$ | |

F) PBS-Puffer (pH 7,0)

| | |
|---|---|
| NaCl | 8,5 g |
| $Na_2HPO_4 \cdot 2H_2O$ | 1,28 g |
| $NaH_2PO_4 \cdot 2H_2O$ | 0,436 g |
| ad 1000 ml $H_2O$ | |

G) PBS-Tween-20 (0,1 %)

1 ml Tween-20 (Serva) + 1000 ml PBS

H) PBS-BSA (1 %)

| | |
|---|---|
| BSA | 5 g |
| $NaN_3$ (0,5M) | 3 ml |
| ad 500 ml PBS | |

20

I) Substrat-Puffer (Diethanolamin-Puffer, pH 9,8)

Diethanolamin 97 ml
NaN$_3$ (0,5M) 6 ml
MgCl$_2$·6H$_2$O 100 mg
ad 1000 ml H$_2$O, Einstellen des pH-Wertes auf pH 9,8 mit HCl conc. Herstellung des Substrates: Unmittelbar vor Gebrauch wird eine Substrattablette (=5 mg) des p-Nitrophenylphosphat-Substrates (Sigma-104) in 5 ml Substratpuffer gelöst.

EP 0 365 818 B1

Tabelle 1: Kreuzreaktivität verschiedener s-Triazin und Hydroxytriazin Analoger mit einem Anti-Atrazin MAb (MAb 4063-21-1) und zwei Anti-Hydroxyatrazin MAb (MAb 4009-85-3 and MAb 4009-77-20)

| Inhibitor | MAb 4063-21-1 (ECACC 8808/2501) | | MAb 4009-85-3 (ECACC 8808/2503) | | Mab 4009-77-20 (ECACC 8808/2502) | |
|---|---|---|---|---|---|---|
| | I 50 ng/ml (a) | % cross-reactivity (b) | I 50 ng/ml (a) | % cross-reactivity (c) | I 50 ng/ml (a) | % cross-reactivity (c) |
| Atrazin | 0,45 | 100,0 | >1000 | <0,1 | >1000 | <0,05 |
| Propazin | 0,5 | 90,0 | >1000 | <0,1 | >1000 | <0,05 |
| Simazin | 18 | 2,5 | | | | |
| Desmetryn | 90 | 0,5 | | | | |
| Terbuthylazin | 9 | 5,0 | | | | |
| Trietazin | 4 | 11,3 | | | | |
| Ametryn | 9 | 5,0 | >1000 | <0,1 | >1000 | <0,05 |
| Atraton | 7 | 6,4 | >1000 | <0,1 | >1000 | <0,05 |
| Prometryn | 0,8 | 56,3 | | | | |
| Simetryn | 27 | 1,7 | | | | |
| Prometon | 0,6 | 75,0 | | | | |
| OH-Atrazin | 21 | 2,1 | 0,95 | 100,0 | 0,5 | 100,0 |
| OH-Propazin | 5,5 | 8,2 | 0,95 | 100,0 | 0,6 | 83,3 |
| OH-Simazin | 180 | 0,3 | 65 | 1,5 | 1,2 | 41,7 |
| OH-Desmetryn | 360 | 0,1 | 8 | 11,9 | 0,7 | 71,4 |
| OH-Terbuthylazin | 8 | 5,6 | 19 | 5,0 | 3,3 | 15,2 |

a) Inhibitorkonzentration für 50 %ige Hemmung im kompetitiven ELISA-Test

b) Kreuzreaktivität, definiert als: (Atrazinkonzentration für 50 %ige Hemmung)/(Konzentration der s-Triazinanalogen für 50 %ige Hemmung) x 100

c) Kreuzreaktivität, definiert als: (Hydroxyatrazinkonzentration für 50 %ige Hemmung)/(Konzentration der s-Triazinanalogen für 50 %ige Hemmung) x 100

Tabelle II. Rückgewinnung von Hydroxyatrazin aus zuvor mit Hydroxyatrazin geimpften Bodenextrakten*

| Erdprobe | Bodenzusammensetzung | | | | pH | Hydroxyatrazin Zugabe (ppb) | Hydroxyatrazin Rückgewinnungsrate (%) MAb 4009-85-3 (ECACC 8808/2503) |
|---|---|---|---|---|---|---|---|
| | % Humus | % Sand | % Schlick | % Lehm | | | |
| Risilon (Israel) | 1,8 | 80,8 | 3,8 | 13,6 | 7,8 | 20 | 91 |
| | | | | | | 60 | 92 |
| | | | | | | 200 | 90 |
| | | | | | | 600 | 93 |
| Vetroz (Schweiz) | 9,3 | 18,1 | 60,4 | 21,5 | 7,3 | 100 | 97 |
| Stein (Schweiz) | 5 | 43 | 17,4 | 34,6 | 7,1 | 100 | 110 |
| Collombey (Schweiz) | 1,4 | 83,9 | 13,6 | 2,5 | 7,4 | 100 | 107 |
| Les Evouettes (Schweiz) | 2,6 | 25,7 | 64 | 10,3 | 6,2 | 100 | 94 |

*angereicherte Bodenextrakte (Methanol-Extrakte) werden 1:40 in PBS-Tween (0,1 %) für die ELISA Bestimmung verdünnt.

Tabelle III.  Bestimmung von Atrazin in Wasserproben
Vergleich zwischen HPLC und ELISA (MAb 4063-21-1; ECACC 8808/2501)

| Wasserprobe | HPLC | | ELISA | Verhältnis |
| | Atrazin µg/l | Desethylatrazin µg/l | s-Triazin µg/l | ELISA/HPLC |
|---|---|---|---|---|
| 1 | 0,09 | 0,05 | 0,10 | 1,1 |
| 2 | 0,10 | 0,15 | 0,14 | 1,4 |
| 3 | 0,41 | 0,59 | 0,38 | 0,9 |
| 4 | 0,10 | 0,11 | 0,15 | 1,5 |
| 5 | 0,08 | 0,06 | 0,11 | 1,4 |
| 6 | 0,06 | 0,05 | 0,09 | 1,5 |
| 7 | <0,05 | 0,11 | 0,07 | >1,0 |
| 8 | <0,05 | 0,05 | 0,06 | >1,0 |
| 9 | 0,06 | <0,05 | 0,07 | 1,2 |
| 10 | <0,05 | <0,05 | 0,07 | >1,0 |
| 11 | <0,05 | <0,05 | 0,08 | >1,0 |
| 12 | 0,14 | 0,29 | 0,10 | 0,7 |
| 13 | 0,23 | 0,14 | 0,19 | 0,8 |
| 14 | 0,23 | 0,13 | 0,34 | 1,5 |
| 15 | 0,23 | 0,13 | 0,24 | 1,0 |
| 16 | 0,36 | 0,24 | 0,49 | 1,4 |
| 17 | 0,35 | 0,23 | 0,43 | 1,2 |
| 18 | 0,40 | 0,24 | 0,33 | 0,8 |
| 19 | 0,40 | 0,27 | 0,41 | 1,0 |
| 20 | 0,50 | 0,32 | 0,50 | 1,0 |
| 21 | 0,45 | 0,29 | 0,54 | 1,2 |
| Kontrolle H$_2$O (HPLC) | | | 0,01 | |

Literatur

Ercegovich CD et al, J. Agric. Food Chem., 29: 559-563, 1981
Fleeker J, J. Assoc. Off. Anal. Chem., 70: 874-878, 1987
Hsu HT et al, ASM News, 50 (3): 91-101, 1984
Kawamura H, Berzojsky JA, J. Immunol., 136: 58, 1986
Kelley M et al, J. Agric. Food Chem., 33: 962-965, 1985
Köhler G, Milstein, Nature, 256: 495-497, 1975
Kulkarni NP et al, Cancer Res., 41: 2700-2706, 1981

24

Littlefield JW, Science, 145: 709, 1964
Newsome WH, J. Agric. Food Chem., 33: 528-530, 1985
Raab GM, Clin. Chem., 29: 1757-1761, 1983
Ramsteiner KA, Hörman WD, J. Agric. Food Chem., 27: 934-938, 1979
Ramsteiner K, "Atrazin". In Methodensammlung zur Rückstandsanalytik von Pflanzenschutzmitteln; Deutsche Forschungsgemeinschaft Ed.; Verlag Chemie, 1985; Methode 6-D
Shulman M et al, Nature, 276: 269-270, 1978.
Wie SI, Hammock BD, J. Agric. Food Chem., 30: 949-957, 1982

Patentliteratur

US-P 4,530,786

**Patentansprüche**

**Patentansprüche für folgende Vertragsstaaten : AT, BE, CH, DE, FR, GB, GR, IT, LI, LU, NL, SE**

1. Eine Hybridomazellinie, die einen monoklonalen Antikörper produziert, der eine hohe Spezifität und Affinität gegenüber Atrazin aufweist und der eine ausgeprägte Kreuzreaktivität mit strukturell ähnlichen Atrazinderivaten ausgewählt aus der Gruppe bestehend aus Propazin, Prometryn und Prometon zeigt, der aber im wesentlichen keine Kreuzreaktivität mit anderen Atrazinderivaten, insbesondere aber mit Ametryn und Atraton, oder mit Hydroxyanalogen von Atrazin und/oder Atrazinderivaten, ausgewählt aus der Gruppe bestehend aus Hydroxyatrazin, Hydroxypropazin, Hydroxysimazin, Hydroxydesmetryn und Hydroxyterbuthylazin, aufweist.

2. Eine Hybridomazellinie gemäss Anspruch 1, hinterlegt unter der Nummer ECACC 8808/2501.

3. Eine Hybridomazellinie, die einen monoklonalen Antikörper produziert, der eine hohe Spezifität und Affinität gegenüber Hydroxyatrazin aufweist und der im wesentlichen keine Kreuzreaktivität mit Atrazin und/oder Atrazinderivaten, ausgewählt aus der Gruppe bestehend aus Propazin, Ametryn und Atraton, zeigt.

4. Eine Hybridomazellinie gemäss Anspruch 3, die einen monoklonalen Antikörper produziert, der eine hohe Spezifität und Affinität gegenüber Hydroxyatrazin aufweist und der eine stark ausgeprägte Kreuzreaktivität mit Hydroxypropazin zeigt, der aber im wesentlichen keine Kreuzreaktivität mit Hydroxyanalogen anderer Atrazinderivate, ausgewählt aus der Gruppe bestehend aus Hydroxysimazin, Hydroxydesmetryn und Hydroxyterbutylazin, oder mit Atrazin und/oder Atrazinderivaten, ausgewählt aus der Gruppe bestehend aus Propazin, Ametryn und Atraton, zeigt.

5. Eine Hybridomazellinie gemäss Anspruch 3, die einen monoklonalen Antikörper produziert, der eine hohe Spezifität und Affinität gegenüber Hydroxyatrazin aufweist und der eine Kreuzreaktivität mit Hydroxyanalogen von Atrazinderivaten, ausgewählt aus der Gruppe bestehend aus Hydroxypropazin, Hydroxysimazin und Hydroxydesmetryn, zeigt, der aber im wesentlichen keine Kreuzreaktivität mit Atrazin und/oder Atrazinderivaten, ausgewählt aus der Gruppe bestehend aus Propazin, Ametryn und Atraton, zeigt.

6. Hybridomazellinie gemäss Anspruch 4, hinterlegt unter der Nummer ECACC 8808/2503.

7. Hybridomazellinie gemäss Anspruch 5, hinterlegt unter der Nummer ECACC 8808/2502. besitzt.

8. Mutanten und Varianten einer Hybridomazellinie gemäss einem der Ansprüche 1 bis 7, die noch die charakteristischen Eigenschaften des Ausgangsmaterials aufweisen; d.h. die noch in der Lage sind die erfindungsgemässen Antikörper zu synthetisieren und ins umgebende Medium zu sezernieren.

9. Monoklonale Antikörper und deren Derivate, die eine hohe Spezifität und Affinität gegenüber Atrazin aufweisen und die eine ausgeprägte Kreuzreaktivität mit strukturell ähnlichen Atrazinderivaten ausgewählt aus der Gruppe bestehend aus Propazin, Prometryn und Prometon zeigen, die aber im wesentlichen keine Kreuzreaktivität mit anderen Atrazinderivaten, insbesondere aber mit Ametryn und Atraton, oder mit Hydroxyanalogen von Atrazin und/oder Atrazinderivaten, ausgewählt aus der Gruppe bestehend aus

EP 0 365 818 B1

Hydroxyatrazin, Hydroxypropazin, Hydroxysimazin, Hydroxydesmetryn und Hydroxyterbuthylazin, aufweisen.

**10.** Monoklonale Antikörper und deren Derivate gemäss Anspruch 9, dadurch gekennzeichnet, dass die Kreuzreaktivität mit Propazin mehr als 80 % beträgt.

**11.** Monoklonaler Antikörper gemäss Anspruch 10 erhältlich aus einer Hybridomazellinie gemäss Anspruch 2.

**12.** Monoklonale Antikörper und deren Derivate, die eine hohe Spezifität und Affinität gegenüber Hydroxyatrazin aufweisen und die im wesentlichen keine Kreuzreaktivität mit Atrazin und/oder Atrazinderivaten, ausgewählt aus der Gruppe bestehend aus Propazin, Ametryn und Atraton, zeigen.

**13.** Monoklonale Antikörper und deren Derivate gemäss Anspruch 12, die eine hohe Spezifität und Affinität gegenüber Hydroxyatrazin aufweisen und die eine stark ausgeprägte Kreuzreaktivität mit Hydroxypropazin zeigen, die aber im wesentlichen keine Kreuzreaktivität mit Hydroxyanalogen anderer Atrazinderivate, ausgewählt aus der Gruppe bestehend aus Hydroxysimazin, Hydroxydesmetryn und Hydroxyterbutylazin, oder mit Atrazin und/oder Atrazinderivaten, ausgewählt aus der Gruppe bestehend aus Propazin, Ametryn und Atraton, zeigen.

**14.** Monoklonale Antikörper und deren Derivate gemäss Anspruch 12, die eine hohe Spezifität und Affinität gegenüber Hydroxyatrazin aufweisen und die eine Kreuzreaktivität mit Hydroxyanalogen von Atrazinderivaten, ausgewählt aus der Gruppe bestehend aus Hydroxypropazin, Hydroxysimazin und Hydroxydesmetryn, aber im wesentlichen keine Kreuzreaktivität mit Atrazin und/oder Atrazinderivaten, ausgewählt aus der Gruppe bestehend aus Propazin, Ametryn und Atraton, zeigen.

**15.** Monoklonale Antikörper und deren Derivate gemäss Anspruch 13, dadurch gekennzeichnet, dass die Kreuzreaktivität mit Hydroxypropazin bis zu 100 % beträgt.

**16.** Monoklonale Antikörper und deren Derivate gemäss Anspruch 14, dadurch gekennzeichnet, dass die Kreuzreaktivität mit Hydroxyanalogen von Atrazinderivaten, ausgewählt aus der Gruppe bestehend aus Hydroxypropazin, Hydroxysimazin und Hydroxydesmetryn, wenigstens 40 % beträgt.

**17.** Monoklonaler Antikörper gemäss Anspruch 15 erhältlich aus einer Hybridomazellinie gemäss Anspruch 6.

**18.** Monoklonaler Antikörper gemäss Anspruch 16 erhältlich aus einer Hybridomazellinie gemäss Anspruch 7.

**19.** Verfahren zur Herstellung einer Hybridomazellinie gemäss einem der Ansprüche 1 bis 7, dadurch gekennzeichnet, dass man
a) Atrazin und/oder ein Atrazinderivat oder Hydroxyatrazin und/oder Hydroxyanaloge von Atrazinderivaten mit einem Carriermolekül konjugiert
b) ein Donor-Tier mit besagtem Konjugat immunisiert
c) eine immunkompetente B-Zelle aus dem immunisierten Donor-Tier isoliert
d) besagte immunkompetente B-Zelle mit einer zur kontinuierlichen Zellteilung befähigten Tumorzellinie fusioniert
e) das entstehende Fusionsprodukt isoliert, in einem geeigneten Kulturmedium kultiviert und anschliessend kloniert und
f) die klonierten Hybridzellen auf die Bildung monoklonaler Antikörper hin untersucht.

**20.** Verfahren gemäss Anspruch 19, dadurch gekennzeichnet, dass man als Carriermolekül makromolekulare Verbindungen verwendet, die frei zugängliche reaktive Gruppen für die Kupplungsreaktion mit Atrazin und/oder Atrazinderivaten oder Hydroxyatrazin und/oder Hydroxyanalogen von Atrazinderivaten aufweisen und die in der Lage sind besagten Verbindungen eine immunogene Potenz zu verleihen.

**21.** Verfahren gemäss Anspruch 20, dadurch gekennzeichnet, dass man ein lysinreiches Protein mit einem Molekulargewicht zwischen 10'000 und 1'500'000 verwendet.

**22.** Verfahren gemäss Anspruch 19, dadurch gekennzeichnet, dass die Konjugation an das Carriermolekül

26

über ein Brückenglied (Spacer) erfolgt, das eine oder mehrere reaktive Gruppen besitzt, die in der Lage sind mit den reaktiven Gruppen des Carriermoleküls in Wechselwirkung zu treten.

23. Verfahren gemäss Anspruch 22, dadurch gekennzeichnet, dass es sich bei besagten reaktiven Gruppen um Carboxyl-, Amino- oder SH-Gruppen handelt.

24. Verfahren gemäss Anspruch 19, dadurch gekennzeichnet, dass die Kupplungsreaktion mit Hilfe der aktiven Estermethode durchgeführt wird.

25. Verfahren gemäss Anspruch 26, dadurch gekennzeichnet, dass man als Fusionsmedium eine Pufferlösung verwendet, die eines der üblicherweise für die Fusionierung von Zellen verwendeten Fusionspromotoren enthält ausgewählt aus der Gruppe bestehend aus: Sendai Viren oder andere Paramyxoviren, Calcium-Ionen, oberflächenaktive Lipide oder Polyethylenglykol.

26. Verfahren gemäss Anspruch 25, dadurch gekennzeichnet, dass es sich bei besagten Fusionspromotoren um Polyethylenglykol mit einem mittleren Molekulargewicht von 600 bis 6000 handelt, dessen Konzentration im Fusionssmedium 30 % - 60 % beträgt.

27. Verfahren gemäss Anspruch 19, dadurch gekennzeichnet, dass man die Hybridzellen in Gegenwart von isolierten Makrophagen ("Feederzellen") kultiviert.

28. Verfahren gemäss Anspruch 19, dadurch gekennzeichnet, dass man positive, monoklonale Antikörper produzierende Hybridzellkulturen mit Hilfe des limitierenden Verdünnungsverfahrens vereinzelt und anschliessend in geeigneten Kultivierungsmedien kloniert.

29. Verfahren gemäss Anspruch 19, dadurch gekennzeichnet, dass man die gemäss Anspruch 28 klonierten Hybridoma-Zellklone mit Hilfe eines Immunoassays auf die Bildung geeigneter monoklonaler Antikörper hin untersucht.

30. Verfahren zur Herstellung monoklonaler Antikörper, dadurch gekennzeichnet, dass man die nach einem Verfahren gemäss einem der Ansprüche 19 bis 29 hergestellten Hybridomazellinien _in vivo_ oder _in vitro_ mit Hilfe bekannter Verfahren kultiviert und die produzierten monoklonalen Antikörper isoliert.

31. Verfahren gemäss Anspruch 30, dadurch gekennzeichnet, dass man besagte monoklonale Antikörper durch Expansion der nach einem Verfahren gemäss einem der Ansprüche 19 bis 29 hergestellten Hybridomazellinien in einem Donor-Tier _in vivo_ herstellt.

32. Verfahren zum immunologischen Nachweis von Atrazin und/oder Atrazinderivaten, dadurch gekennzeichnet, dass man einen monoklonalen Antikörper gemäss einem der Ansprüche 9 oder 10 in einem der bekannten Immunassays verwendet.

33. Verfahren zum immunologischen Nachweis von Atrazin und/oder Atrazinderivaten gemäss Anspruch 32, dadurch gekennzeichnet, dass man einen monoklonalen Antikörper, der von einer Hybridomazellinie gemäss Anspruch 2 produziert wird, oder von Klonen oder Subklonen davon, in einem der bekannten Immunassays verwendet.

34. Verfahren zum immunologischen Nachweis von Hydroxyatrazin und/oder Hydroxyatrazinderivaten, dadurch gekennzeichnet, dass man einen monoklonalen Antikörper gemäss einem der Ansprüche 12 bis 16 in einem der bekannten Immunassays verwendet.

35. Verfahren zum immunologischen Nachweis von Hydroxyatrazin und/oder Hydroxyatrazinderivaten gemäss Anspruch 34, dadurch gekennzeichnet, dass man einen monoklonalen Antikörper, der von einer Hybridomazellinie gemäss Anspruch 6, oder von Klonen oder Subklonen davon, produziert wird, in einem der bekannten Immunassays verwendet.

36. Verfahren zum immunologischen Nachweis von Hydroxyatrazin und/oder Hydroxyatrazinderivaten gemäss Anspruch 34, dadurch gekennzeichnet, dass man einen monoklonalen Antikörper, der von einer Hybridomazellinie gemäss Anspruch 7, oder von Klonen oder Subklonen davon, produziert wird, in einem der bekannten Immunassays verwendet.

EP 0 365 818 B1

37. Verfahren gemäss einem der Ansprüche 32 bis 36, dadurch gekennzeichnet, dass es sich um einen kompetitiven Immunassay handelt.

38. Verfahren gemäss einem der Ansprüche 32 bis 37, dadurch gekennzeichnet, dass es sich bei besagtem Immunassay um einen Radioimmunassay (RIA), einen enzymgekoppelten Assay (ELISA) oder einen Chemolumineszenzassay handelt.

39. Mittel zum immunologischen Nachweis von Atrazin und/oder Atrazinderivaten in Form eines gebrauchsfertigen Test-Kits, dadurch gekennzeichnet, dass besagter Test-Kit neben den üblicherweise verwendeten Trägermaterialien, Reagentien und sonstigen Zusätzen mindestens einen monoklonalen Antikörper gemäss einem der Ansprüche 9 oder 10 als Reagenz enthält.

40. Mittel zum immunologischen Nachweis von Atrazin und/oder Atrazinderivaten in Form eines gebrauchsfertigen Test-Kits gemäss Anspruch 39, dadurch gekennzeichnet, dass besagter Test-Kit neben den üblicherweise verwendeten Trägermaterialien, Reagentien und sonstigen Zusätzen mindestens einen monoklonalen Antikörper enthält, der von einer Hybridomazellinie gemäss Anspruch 2, oder von Klonen oder Subklonen davon, produziert wird.

41. Mittel zum immunologischen Nachweis von Hydroxyatrazin und/oder Hydroxyanalogen von Atrazinderivaten in Form eines gebrauchsfertigen Test-Kits, dadurch gekennzeichnet, dass besagter Testkit neben den üblicherweise verwendeten Trägermaterialien, Reagentien und sonstigen Zusätzen mindestens einen monoklonalen Antikörper gemäss einem der Ansprüche 12 bis 16 als Reagenz enthält.

42. Mittel zum immunologischen Nachweis von Hydroxyatrazin und/oder Hydroxyanalogen von Atrazinderivaten in Form eines gebrauchsfertigen Test-Kits gemäss Anspruch 41, dadurch gekennzeichnet, dass besagter Test-Kit neben den üblicherweise verwendeten Trägermaterialien, Reagentien und sonstigen Zusätzen mindestens einen monoklonalen Antikörper als Reagenz enthält, der von einer Hybridomazellinie gemäss Anspruch 6, oder von Klonen oder Subklonen davon, produziert wird.

43. Mittel zum immunologischen Nachweis von Hydroxyatrazin und/oder Hydroxyanalogen von Atrazinderivaten in Form eines gebrauchsfertigen Test-Kits gemäss Anspruch 41, dadurch gekennzeichnet, dass besagter Test-Kit neben den üblicherweise verwendeten Trägermaterialien, Reagentien und sonstigen Zusätzen mindestens einen monoklonalen Antikörper als Reagenz enthält, der von einer Hybridomazellinie gemäss Anspruch 7, oder von Klonen oder Subklonen davon, produziert wird.

**Patentansprüche für folgenden Vertragsstaat : ES**

1. Verfahren zur Herstellung einer Hybridomazellinie, die einen monoklonalen Antikörper produziert, der eine hohe Spezifität und Affinität gegenüber Atrazin aufweist und der eine ausgeprägte Kreuzreaktivität mit strukturell ähnlichen Atrazinderivaten ausgewählt aus der Gruppe bestehend aus Propazin, Prometryn und Prometon zeigt, der aber im wesentlichen keine Kreuzreaktivität mit anderen Atrazinderivaten, insbesondere aber mit Ametryn und Atraton, oder mit Hydroxyanalogen von Atrazin und/oder Atrazinderivaten, ausgewählt aus der Gruppe bestehend aus Hydroxyatrazin, Hydroxypropazin, Hydroxysimazin, Hydroxydesmetryn und Hydroxyterbuthylazin, aufweist, dadurch gekennzeichnet, dass man
   a) Atrazin und/oder ein Atrazinderivat mit einem Carriermolekül konjugiert
   b) ein Donor-Tier mit besagtem Konjugat immunisiert
   c) eine immunkompetente B-Zelle aus dem immunisierten Donor-Tier isoliert
   d) besagte immunkompetente B-Zelle mit einer zur kontinuierlichen Zellteilung befähigten Tumorzellinie fusioniert
   e) das entstehende Fusionsprodukt isoliert, in einem geeigneten Kulturmedium kultiviert und anschliessend kloniert und
   f) die klonierten Hybridzellen auf die Bildung monoklonaler Antikörper hin untersucht.

2. Verfahren gemäss Anspruch 1, dadurch gekennzeichnet, dass es sich bei besagter Hybridomazellinie um die unter der Nummer ECACC 8808/2501 hinterlegte Zellinie handelt.

3. Verfahren zur Herstellung einer Hybridomazellinie, die einen monoklonalen Antikörper produziert, der eine hohe Spezifität und Affinität gegenüber Hydroxyatrazin aufweist und der im wesentlichen keine Kreuzreaktivität mit Atrazin und/oder Atrazinderivaten, ausgewählt aus der Gruppe bestehend aus Propazin,

28

Ametryn und Atraton, zeigt, dadurch gekennzeichnet, dass man

a) Hydroxyatrazin und/oder Hydroxyanaloge von Atrazinderivaten mit einem Carriermolekül konjugiert

b) ein Donor-Tier mit besagtem Konjugat immunisiert

c) eine immunkompetente B-Zelle aus dem immunisierten Donor-Tier isoliert

d) besagte immunkompetente B-Zelle mit einer zur kontinuierlichen Zellteilung befähigten Tumorzellinie fusioniert

e) das entstehende Fusionsprodukt isoliert, in einem geeigneten Kulturmedium kultiviert und anschliessend kloniert und

f) die klonierten Hybridzellen auf die Bildung monoklonaler Antikörper hin untersucht.

4. Verfahren gemäss Anspruch 3, dadurch gekennzeichnet, dass besagte Hybridomazellinie einen monoklonalen Antikörper produziert, der eine hohe Spezifität und Affinität gegenüber Hydroxyatrazin aufweist und der eine stark ausgeprägte Kreuzreaktivität mit Hydroxypropazin zeigt, der aber im wesentlichen keine Kreuzreaktivität mit Hydroxyanalogen anderer Atrazinderivate, ausgewählt aus der Gruppe bestehend aus Hydroxysimazin, Hydroxydesmetryn und Hydroxyterbutylazin, oder mit Atrazin und/oder Atrazinderivaten, ausgewählt aus der Gruppe bestehend aus Propazin, Ametryn und Atraton, zeigt.

5. Verfahren gemäss Anspruch 3, dadurch gekennzeichnet, dass besagte Hybridomazellinie einen monoklonalen Antikörper produziert, der eine hohe Spezifität und Affinität gegenüber Hydroxyatrazin aufweist und der eine Kreuzreaktivität mit Hydroxyanalogen von Atrazinderivaten, ausgewählt aus der Gruppe bestehend aus Hydroxypropazin, Hydroxysimazin und Hydroxydesmetryn, zeigt, der aber im wesentlichen keine Kreuzreaktivität mit Atrazin und/oder Atrazinderivaten, ausgewählt aus der Gruppe bestehend aus Propazin, Ametryn und Atraton, zeigt.

6. Verfahren gemäss Anspruch 4, dadurch gekennzeichnet, dass es sich bei besagter Hybridomazellinie um die unter der Nummer ECACC 8808/2503 hinterlegte Zellinie handelt.

7. Verfahren gemäss Anspruch 5, dadurch gekennzeichnet, dass es sich bei besagter Hybridomazellinie um die unter der Nummer ECACC 8808/2502 hinterlegte Zellinie handelt.

8. Verfahren gemäss einem der Ansprüche 1 bis 7, dadurch gekennzeichnet, dass man als Carriermolekül makromolekulare Verbindungen verwendet, die frei zugängliche reaktive Gruppen für die Kupplungsreaktion mit Atrazin und/oder Atrazinderivaten oder Hydroxyatrazin und/oder Hydroxyanalogen von Atrazinderivaten aufweisen und die in der Lage sind besagten Verbindungen eine immunogene Potenz zu verleihen.

9. Verfahren gemäss Anspruch 8, dadurch gekennzeichnet, dass man ein lysinreiches Protein mit einem Molekulargewicht zwischen 10'000 und 1'500'000 verwendet.

10. Verfahren gemäss einem der Ansprüche 1 bis 7, dadurch gekennzeichnet, dass die Konjugation an das Carriermolekül über ein Brückenglied (Spacer) erfolgt, das eine oder mehrere reaktive Gruppen besitzt, die in der Lage sind mit den reaktiven Gruppen des Carriermoleküls in Wechselwirkung zu treten.

11. Verfahren gemäss Anspruch 10, dadurch gekennzeichnet, dass es sich bei besagten reaktiven Gruppen um Carboxyl-, Amino- oder SH-Gruppen handelt.

12. Verfahren gemäss einem der Ansprüche 1 bis 7, dadurch gekennzeichnet, dass die Kupplungsreaktion mit Hilfe der aktiven Estermethode durchgeführt wird.

13. Verfahren gemäss einem der Ansprüche 1 bis 7, dadurch gekennzeichnet, dass man als Fusionsmedium eine Pufferlösung verwendet, die eines der üblicherweise für die Fusionierung von Zellen verwendeten Fusionspromotoren enthält ausgewählt aus der Gruppe bestehend aus: Sendai Viren oder andere Paramyxoviren, Calcium-Ionen, oberflächenaktive Lipide oder Polyethylenglykol.

14. Verfahren gemäss Anspruch 13, dadurch gekennzeichnet, dass es sich bei besagten Fusionspromotoren um Polyethylenglykol mit einem mittleren Molekulargewicht von 600 bis 6000 handelt, dessen Konzentration im Fusionssmedium 30 % - 60 % beträgt.

15. Verfahren gemäss einem der Ansprüche 1 bis 7, dadurch gekennzeichnet, dass man die Hybridzellen in Gegenwart von isolierten Makrophagen ("Feederzellen") kultiviert.

16. Verfahren gemäss einem der Ansprüche 1 bis 7, dadurch gekennzeichnet, dass man positive, monoklonale Antikörper produzierende Hybridzellkulturen mit Hilfe des limitierenden Verdünnungsverfahrens vereinzelt und anschliessend in geeigneten Kultivierungsmedien kloniert.

17. Verfahren gemäss einem der Ansprüche 1 bis 7, dadurch gekennzeichnet, dass man die gemäss Anspruch 16 klonierten Hybridoma-Zellklone mit Hilfe eines Immunoassays auf die Bildung geeigneter monoklonaler Antikörper hin untersucht.

18. Verfahren zur Herstellung von Mutanten und Varianten einer Hybridomazellinie gemäss einem der Ansprüche 1 bis 7, dadurch gekennzeichnet, dass man besagte Hybridomzellinie mit Hilfe an sich bekannter Verfahren mutiert und diejenigen Hybridomazellinien selektiert, welche noch die charakteristischen Eigenschaften des Ausgangsmaterials aufweisen; d.h. die noch in der Lage sind die erfindungsgemässen Antikörper zu synthetisieren und ins umgebende Medium zu sezernieren.

19. Verfahren zur Herstellung monoklonaler Antikörper und deren Derivate, die eine hohe Spezifität und Affinität gegenüber Atrazin aufweisen und die eine ausgeprägte Kreuzreaktivität mit strukturell ähnlichen Atrazinderivaten ausgewählt aus der Gruppe bestehend aus Propazin, Prometryn und Prometon zeigen, die aber im wesentlichen keine Kreuzreaktivität mit anderen Atrazinderivaten, insbesondere aber mit Ametryn und Atraton, oder mit Hydroxyanalogen von Atrazin und/oder Atrazinderivaten, ausgewählt aus der Gruppe bestehend aus Hydroxyatrazin, Hydroxypropazin, Hydroxysimazin, Hydroxydesmetryn und Hydroxyterbuthylazin, aufweisen, dadurch gekennzeichnet, dass man die nach einem Verfahren gemäss Anspruch 1 hergestellte Hybridomazellinie in vivo oder in vitro mit Hilfe bekannter Verfahren kultiviert und die produzierten monoklonalen Antikörper isoliert.

20. Verfahren gemäss Anspruch 19, dadurch gekennzeichnet, dass die Kreuzreaktivität besagter monoklonaler Antikörper mit Propazin mehr als 80 % beträgt.

21. Verfahren gemäss Anspruch 19, dadurch gekennzeichnet, dass es sich bei besagter Hybridomazellinie um die unter der Nummer ECACC 8808/2501 hinterlegte Zellinie handelt.

22. Verfahren zur Herstellung monoklonaler Antikörper und deren Derivate, die eine hohe Spezifität und Affinität gegenüber Hydroxyatrazin aufweisen und die im wesentlichen keine Kreuzreaktivität mit Atrazin und/oder Atrazinderivaten, ausgewählt aus der Gruppe bestehend aus Propazin, Ametryn und Atraton, zeigen, dadurch gekennzeichnet, dass man die nach einem Verfahren gemäss Anspruch 3 hergestellte Hybridomazellinie in vivo oder in vitro mit Hilfe bekannter Verfahren kultiviert und die produzierten monoklonalen Antikörper isoliert.

23. Verfahren gemäss Anspruch 22, dadurch gekennzeichnet, dass besagte monoklonale Antikörper eine hohe Spezifität und Affinität gegenüber Hydroxyatrazin aufweisen und eine stark ausgeprägte Kreuzreaktivität mit Hydroxypropazin zeigen, im wesentlichen aber keine Kreuzreaktivität mit Hydroxyanalogen anderer Atrazinderivate, ausgewählt aus der Gruppe bestehend aus Hydroxysimazin, Hydroxydesmetryn und Hydroxyterbutylazin, oder mit Atrazin und/oder Atrazinderivaten, ausgewählt aus der Gruppe bestehend aus Propazin, Ametryn und Atraton, zeigen.

24. Verfahren gemäss Anspruch 22, dadurch gekennzeichnet, dass besagte monoklonale Antikörper eine hohe Spezifität und Affinität gegenüber Hydroxyatrazin aufweisen und eine Kreuzreaktivität mit Hydroxyanalogen von Atrazinderivaten, ausgewählt aus der Gruppe bestehend aus Hydroxypropazin, Hydroxysimazin und Hydroxydesmetryn, im wesentlichen aber keine Kreuzreaktivität mit Atrazin und/oder Atrazinderivaten, ausgewählt aus der Gruppe bestehend aus Propazin, Ametryn und Atraton, zeigen.

25. Verfahren gemäss Anspruch 23, dadurch gekennzeichnet, dass die Kreuzreaktivität mit Hydroxypropazin bis zu 100 % beträgt.

26. Verfahren gemäss Anspruch 24, dadurch gekennzeichnet, dass die Kreuzreaktivität mit Hydroxyanalogen von Atrazinderivaten, ausgewählt aus der Gruppe bestehend aus Hydroxypropazin, Hydroxysimazin und Hydroxydesmetryn, wenigstens 40 % beträgt.

27. Verfahren gemäss Anspruch 25, dadurch gekennzeichnet, dass es sich bei besagter Hybridomazellinie um die unter der Nummer ECACC 8808/2503 hinterlegte Zellinie handelt.

**28.** Verfahren gemäss Anspruch 26, dadurch gekennzeichnet, dass es sich bei besagter Hybridomazellinie um die unter der Nummer ECACC 8808/2502 hinterlegte Zellinie handelt.

**29.** Verfahren gemäss einem der Ansprüche 19 bis 28, dadurch gekennzeichnet, dass man besagte monoklonale Antikörper durch Expansion der Hybridomazellinien in einem Donor-Tier <u>in vivo</u> herstellt.

**30.** Verfahren zum immunologischen Nachweis von Atrazin und/oder Atrazinderivaten, dadurch gekennzeichnet, dass man einen nach einem Verfahren gemäss einem der Ansprüche 19 oder 20 hergestellten monoklonalen Antikörper in einem der bekannten Immunassays verwendet.

**31.** Verfahren zum immunologischen Nachweis von Atrazin und/oder Atrazinderivaten gemäss Anspruch 30, dadurch gekennzeichnet, dass man einen monoklonalen Antikörper, der von einer nach dem Verfahren gemäss Anspruch 2 hergestellten Hybridomazellinie produziert wird, oder von Klonen oder Subklonen davon, in einem der bekannten Immunassays verwendet.

**32.** Verfahren zum immunologischen Nachweis von Hydroxyatrazin und/oder Hydroxyatrazinderivaten, dadurch gekennzeichnet, dass man einen monoklonalen Antikörper gemäss einem der Ansprüche 22 bis 26 in einem der bekannten Immunassays verwendet.

**33.** Verfahren zum immunologischen Nachweis von Hydroxyatrazin und/oder Hydroxyatrazinderivaten gemäss Anspruch 32, dadurch gekennzeichnet, dass man einen monoklonalen Antikörper, der von einer nach einem Verfahren gemäss Anspruch 6 hergestellten Hybridomazellinie, oder von Klonen oder Subklonen davon, produziert wird, in einem der bekannten Immunassays verwendet.

**34.** Verfahren zum immunologischen Nachweis von Hydroxyatrazin und/oder Hydroxyatrazinderivaten gemäss Anspruch 32, dadurch gekennzeichnet, dass man einen monoklonalen Antikörper, der von einer nach einem Verfahren gemäss Anspruch 7 hergestellten Hybridomazellinie, oder von Klonen oder Subklonen davon, produziert wird, in einem der bekannten Immunassays verwendet.

**35.** Verfahren gemäss einem der Ansprüche 30 bis 34, dadurch gekennzeichnet, dass es sich um einen kompetitiven Immunassay handelt.

**36.** Verfahren gemäss einem der Ansprüche 30 bis 35, dadurch gekennzeichnet, dass es sich bei besagtem Immunassay um einen Radioimmunassay (RIA), einen enzymgekoppelten Assay (ELISA) oder einen Chemolumineszenzassay handelt.

**37.** Mittel zum immunologischen Nachweis von Atrazin und/oder Atrazinderivaten in Form eines gebrauchsfertigen Test-Kits, dadurch gekennzeichnet, dass besagter Test-Kit neben den üblicherweise verwendeten Trägermaterialien, Reagentien und sonstigen Zusätzen mindestens einen nach einem Verfahren gemäss einem der Ansprüche 19 oder 20 hergestellten monoklonalen Antikörper als Reagenz enthält.

**38.** Mittel zum immunologischen Nachweis von Atrazin und/oder Atrazinderivaten in Form eines gebrauchsfertigen Test-Kits gemäss Anspruch 37, dadurch gekennzeichnet, dass besagter Test-Kit neben den üblicherweise verwendeten Trägermaterialien, Reagentien und sonstigen Zusätzen mindestens einen monoklonalen Antikörper enthält, der von einer nach einem Verfahren gemäss Anspruch 2 hergestellten Hybridomazellinie, oder von Klonen oder Subklonen davon, produziert wird.

**39.** Mittel zum immunologischen Nachweis von Hydroxyatrazin und/oder Hydroxyanalogen von Atrazinderivaten in Form eines gebrauchsfertigen Test-Kits, dadurch gekennzeichnet, dass besagter Testkit neben den üblicherweise verwendeten Trägermaterialien, Reagentien und sonstigen Zusätzen mindestens einen nach einem Verfahren gemäss einem der Ansprüche 22 bis 26 hergestellten monoklonalen Antikörper als Reagenz enthält.

**40.** Mittel zum immunologischen Nachweis von Hydroxyatrazin und/oder Hydroxyanalogen von Atrazinderivaten in Form eines gebrauchsfertigen Test-Kits gemäss Anspruch 39, dadurch gekennzeichnet, dass besagter Test-Kit neben den üblicherweise verwendeten Trägermaterialien, Reagentien und sonstigen Zusätzen mindestens einen monoklonalen Antikörper als Reagenz enthält, der von einer nach einem Verfahren gemäss Anspruch 6 hergestellten Hybridomazellinie, oder von Klonen oder Subklonen davon, produziert wird.

31

41. Mittel zum immunologischen Nachweis von Hydroxyatrazin und/oder Hydroxyanalogen von Atrazinderivaten in Form eines gebrauchsfertigen Test-Kits gemäss Anspruch 39, dadurch gekennzeichnet, dass besagter Test-Kit neben den üblicherweise verwendeten Trägermaterialien, Reagentien und sonstigen Zusätzen mindestens einen monoklonalen Antikörper als Reagenz enthält, der von einer nach einem Verfahren gemäss Anspruch 7 hergestellten Hybridomazellinie, oder von Klonen oder Subklonen davon, produziert wird.

## Claims

### Claims for the following Contracting States : AT, BE, CH, DE, FR, GB, GR, IT, LI, LU, NL, SE

1. A hybridoma cell line which produces a monoclonal antibody which has a high specificity and affinity for atrazine and which shows a pronounced cross-reactivity with structurally similar atrazine derivatives selected from the group comprising propazine, prometryn and prometon but which has essentially no cross-reactivity with other atrazine derivatives, but especially with ametryn and atraton, or with hydroxyl analogues of atrazine and/or atrazine derivatives, selected from the group comprising hydroxyatrazine, hydroxypropazine, hydroxysimazine, hydroxydesmetryn and hydroxyterbuthylazine.

2. A hybridoma cell line according to claim 1, deposited under the number ECACC 8808/2501.

3. A hybridoma cell line which produces a monoclonal antibody which has a high specificity and affinity for hydroxyatrazine and which shows essentially no cross-reactivity with atrazine and/or atrazine derivatives selected from the group comprising propazine, ametryn and atraton.

4. A hybridoma cell line according to claim 3, which produces a monoclonal antibody which has a high specificity and affinity for hydroxyatrazine and which shows a very pronounced cross-reactivity with hydroxypropazine but which shows essentially no cross-reactivity with hydroxyl analogues of other atrazine derivatives, selected from the group comprising hydroxysimazine, hydroxydesmetryn and hydroxyterbuthylazine, or with atrazine and/or atrazine derivatives selected from the group comprising propazine, ametryn and atraton.

5. A hybridoma cell line according to claim 3, which produces a monoclonal antibody which has a high specificity and affinity for hydroxyatrazine and which shows a cross-reactivity with hydroxyl analogues of atrazine derivatives, selected from the group comprising hydroxypropazine, hydroxysimazine and hydroxydesmetryn, but which shows essentially no cross-reactivity with atrazine and/or atrazine derivatives selected from the group comprising hydroxypropazine, hydroxysimazine and hydroxydesmetryn, but which shows essentially no cross-reactivity with atrazine and/or atrazine derivatives selected from the group comprising propazine, ametryn and atraton.

6. A hybridoma cell line according to claim 4, deposited under the number ECACC 8808/2503.

7. A hybridoma cell line according to claim 5, deposited under the number ECACC 8808/2502.

8. Mutants and variants of a hybridoma cell line according to one of claims 1 to 7, which still have the characteristic properties of the starting material; that is to say are still able to synthesize and to secrete into the surrounding medium the antibodies according to the invention.

9. Monoclonal antibodies and the derivatives thereof which have a high specificity and affinity for atrazine and which show a pronounced cross-reactivity with structurally similar atrazine derivatives selected from the group comprising propazine, prometryn and prometon but which have essentially no cross-reactivity with other atrazine derivatives, but especially with ametryn and atraton, or with hydroxyl analogues of atrazine and/or atrazine derivatives, selected from the group comprising hydroxyatrazine, hydroxypropazine, hydroxysimazine, hydroxydesmetryn and hydroxyterbuthylazine.

10. Monoclonal antibodies and the derivatives thereof according to claim 9, characterized in that the cross-reactivity with propazine is more than 80%.

11. Monoclonal antibodies according to claim 10, obtainable from a hybridoma cell line according to claim 2.

12. Monoclonal antibodies and the derivatives thereof which have a high specificity and affinity for hydroxyatrazine and which show essentially no cross-reactivity with atrazine and/or atrazine derivatives selected from the group comprising propazine, ametryn and atraton.

13. Monoclonal antibodies and the derivatives thereof according to claim 12, which have a high specificity and affinity for hydroxyatrazine and which show a very pronounced cross-reactivity with hydroxypropazine but which show essentially no cross-reactivity with hydroxyl analogues of other atrazine derivatives, selected from the group comprising hydroxysimazine, hydroxydesmetryn and hydroxyterbuthylazine, or with atrazine and/or atrazine derivatives selected from the group comprising propazine, ametryn and atraton.

14. Monoclonal antibodies and the derivatives thereof according to claim 12, which have a high specificity and affinity for hydroxyatrazine and which show a cross-reactivity with hydroxyl analogues of atrazine derivatives, selected from the group comprising hydroxypropazine, hydroxysimazine and hydroxydesmetryn, but which show essentially no cross-reactivity with atrazine and/or atrazine derivatives selected from the group comprising propazine, ametryn and atraton.

15. Monoclonal antibodies and the derivatives thereof according to claim 13, characterized in that the cross-reactivity with hydroxypropazine is up to 100%.

16. Monoclonal antibodies and the derivatives thereof according to claim 14, characterized in that the cross-reactivity with hydroxyl analogues of atrazine derivatives, selected from the group comprising hydroxypropazine, hydroxysimazine and hydroxydesmetryn, is at least 40%.

17. Monoclonal antibody according to claim 15, obtainable from a hybridoma cell line according to claim 6.

18. Monoclonal antibody according to claim 16, obtainable from a hybridoma cell line according to claim 7.

19. Method for the preparation of a hybridoma cell line according to one of claims 1 to 7, characterized in that
a) atrazine and/or an atrazine derivative or hydroxyatrazine and/or hydroxyl analogues of atrazine derivatives are conjugated with a carrier molecule
b) a donor animal is immunized with said conjugate
c) an immunocompetent B cell is isolated from the immunized donor animal
d) said immunocompetent B cell is fused with a tumour cell line which is capable of continuous cell division
e) the resulting fusion product is isolated, cultivated in a suitable culture medium and subsequently cloned and
f) the cloned hybrid cells are investigated for the formation of monoclonal antibodies.

20. Method according to claim 19, characterized in that used as carrier molecule are macromolecular compounds which have reactive groups freely accessible for the coupling reaction with atrazine and/or atrazine derivatives or hydroxyatrazine and/or hydroxyl analogues of atrazine derivatives and which are able to confer an immunogenic potency on said compounds.

21. Method according to claim 20, characterized in that a lysine-rich protein with a molecular weight between 10,000 and 1,500,000 is used.

22. Method according to claim 19, characterized in that the conjugation to the carrier molecule takes place via a bridge member (spacer) which has one or more reactive groups which are able to enter into interaction with the reactive groups of the carrier molecule.

23. Method according to claim 22, characterized in that said reactive groups take the form of carboxyl, amino or SH groups.

24. Method according to claim 19, characterized in that the coupling reaction is carried out by means of the active ester method.

25. Method according to claim 19, characterized in that used as fusion medium is a buffer solution which contains one of the fusion promoters which is customarily used for the fusion of cells and is selected from the group comprising: sendai viruses or other paramyxoviruses, calcium ions, surface-active lipids or polyethylene glycol.

26. Method according to claim 25, characterized in that said fusion promoters take the form of polyethylene glycol with a mean molecular weight of 600 to 6,000 whose concentration in the fusion medium is 30% - 60%.

27. Method according to claim 19, characterized in that the hybrid cells are cultivated in the presence of isolated macrophages ("feeder cells").

28. Method according to claim 19, characterized in that positive hybrid cell cultures producing monoclonal antibodies are singled out using the limiting dilution method and subsequently cloned in suitable cultivation media.

29. Method according to claim 19, characterized in that the hybridoma cell clones cloned according to claim 28 are investigated using an immunoassay for the formation of suitable monoclonal antibodies.

30. Method for the preparation of monoclonal antibodies, characterized in that the hybridoma cell lines prepared according to one of claims 19 to 29 are cultivated in vivo or in vitro using known methods, and the produced monoclonal antibodies are isolated.

31. Method according to claim 30, characterized in that said monoclonal antibodies are prepared by expansion in vivo, in a donor animal, of the hybridoma cell lines prepared according to one of claims 19 to 29.

32. Method for the immunological detection of atrazine and/or atrazine derivatives, characterized in that a monoclonal antibody according to one of claims 9 or 10 is used in one of the known immunoassays.

33. Method for the immunological detection of atrazine and/or atrazine derivatives according to claim 32, characterized in that a monoclonal antibody which is produced by a hybridoma cell line according to claim 2, or by clones or subclones thereof, is used in one of the known immunoassays.

34. Method for the immunological detection of hydroxyatrazine and/or hydroxyatrazine derivatives, characterized in that a monoclonal antibody according to one of claims 12 to 16 is used in one of the known immunoassays.

35. Method for the immunological detection of hydroxyatrazine and/or hydroxyatrazine derivatives according to claim 34, characterized in that a monoclonal antibody which is produced by a hybridoma cell line according to claim 6, or by clones or subclones thereof, is used in one of the known immunoassays.

36. Method for the immunological detection of hydroxyatrazine and/or hydroxyatrazine derivatives according to claim 34, characterized in that a monoclonal antibody which is produced by a hybridoma cell line according to claim 7, or by clones or subclones thereof, is used in one of the known immunoassays.

37. Method according to one of claims 32 to 36, characterized in that it takes the form of a competitive immunoassay.

38. Method according to one of claims 32 to 37, characterized in that the said immunoassay takes the form of a radioimmunoassay (RIA), an enzyme-coupled assay (ELISA) or a chemoluminescence assay.

39. Means for the immunological detection of atrazine and/or atrazine derivatives in the form of a ready-to-use test kit, characterized in that said test kit contains, besides the customarily used carrier materials, reagents and other additives, at least one monoclonal antibody according to one of claims 9 or 10 as reagent.

40. Means for the immunological detection of atrazine and/or atrazine derivatives in the form of a ready-to-use test kit according to claim 39, characterized in that said test kit contains, besides the customarily used carrier materials, reagents and other additives, at least one monoclonal antibody which is produced by a hybridoma cell line according to claim 2, or by clones or subclones thereof.

41. Means for the immunological detection of hydroxyatrazine and/or hydroxyl analogues of atrazine derivatives in the form of a ready-to-use test kit, characterized in that said test kit contains, besides the customarily used carrier materials, reagents and other additives, at least one monoclonal antibody according to

EP 0 365 818 B1

one of claims 12 to 16 as reagent.

42. Means for the immunological detection of hydroxyatrazine and/or hydroxyl analogues of atrazine derivatives in the form of a ready-to-use test kit according to claim 41, characterized in that said test kit contains, besides the customarily used carrier materials, reagents and other additives, at least one monoclonal antibody as reagent which is produced by a hybridoma cell line according to claim 6, or by clones or subclones thereof.

43. Means for the immunological detection of hydroxyatrazine and/or hydroxyl analogues of atrazine derivatives in the form of a ready-to-use test kit according to claim 41, characterized in that said test kit contains, besides the customarily used carrier materials, reagents and other additives, at least one monoclonal antibody as reagent which is produced by a hybridoma cell line according to claim 7, or by clones or subclones thereof.

**Claims for the following Contracting State : ES**

1. A method for the preapration of a hybridoma cell line which produces a monoclonal antibody which has a high specificity and affinity for atrazine and which shows a pronounced cross-reactivity with structurally similar atrazine derivatives selected from the group comprising propazine, prometryn and prometon but which has essentially no cross-reactivity with other atrazine derivatives, but especially with ametryn and atraton, or with hydroxyl analogues of atrazine and/or atrazine derivatives, selected from the group comprising hydroxyatrazine, hydroxypropazine, hydroxysimazine, hydroxydesmetryn and hydroxyterbuthylazine,
characterized in that
   a) atrazine and/or an atrazine derivative is conjugated with a carrier molecule
   b) a donor animal is immunized with said conjugate
   c) an immunocompetent B cell is isolated from the immunized donor animal
   d) said immunocompetent B cell is fused with a tumour cell line which is capable of continuous cell division
   e) the resulting fusion product is isolated, cultivated in a suitable culture medium and subsequently cloned and
   f) the cloned hybrid cells are investigated for the formation of monoclonal antibodies.

2. A method according to claim 1, characterized in that said hybridoma cell line is the cell line deposited under the number ECACC 8808/2501.

3. A method for the preparation of a hybridoma cell line which produces a monoclonal antibody which has a high specificity and affinity for hydroxyatrazine and which shows essentially no cross-reactivity with atrazine and/or atrazine derivatives selected from the group comprising propazine, ametryn and atraton, characterized in that
   a) hydroxyatrazine and/or hydroxyl analogues of atrazine derivatives are conjugated with a carrier molecule
   b) a donor animal is immunized with said conjugate
   c) an immunocompetent B cell is isolated from the immunized donor animal
   d) said immunocompetent B cell is fused with a tumour cell line which is capable of continuous cell division
   e) the resulting fusion product is isolated, cultivated in a suitable culture medium and subsequently cloned and
   f) the cloned hybrid cells are investigated for the formation of monoclonal antibodies.

4. A method according to claim 3, characterized in that said hybridoma cell line produces a monoclonal antibody which has a high specificity and affinity for hydroxyatrazine and which shows a very pronounced cross-reactivity with hydroxypropazine but which shows essentially no cross-reactivity with hydroxyl analogues of other atrazine derivatives, selected from the group comprising hydroxysimazine, hydroxydesmetryn and hydroxyterbuthylazine, or with atrazine and/or atrazine derivatives selected from the group comprising propazine, ametryn and atraton.

5. A method according to claim 3, characterized in that said hybridoma cell line produces a monoclonal antibody which has a high specificity and affinity for hydroxyatrazine and which shows a cross-reactivity with hydroxyl analogues of atrazine derivatives, selected from the group comprising hydroxypropazine, hydroxysimazine and hydroxydesmetryn, but which shows essentially no cross-reactivity with atrazine and/or atrazine derivatives selected from the group comprising propazine, ametryn and atraton.

6. A method according to claim 4, characterized in that said hybridoma cell line is the cell line deposited under the number ECACC 8808/2503.

7. A method according to claim 5, characterized in that said hybridoma cell line is the cell line deposited under the number ECACC 8808/2502.

8. A method according to one of claims 1 to 7, characterized in that used as carrier molecule are macromolecular compounds which have reactive groups freely accessible for the coupling reaction with atrazine and/or atrazine derivatives or hydroxyatrazine and/or hydroxyl analogues of atrazine derivatives and which are able to confer an immunogenic potency on said compounds.

9. A method according to claim 8, characterized in that a lysine-rich protein with a molecular weight between 10,000 and 1,500,000 is used.

10. A method according to one of claims 1 to 7, characterized in that the conjugation to the carrier molecule takes place via a bridge member (spacer) which has one or more reactive groups which are able to enter into interaction with the reactive groups of the carrier molecule.

11. A method according to claim 10, characterized in that said reactive groups take the form of carboxyl, amino or SH groups.

12. A method according to one of claims 1 to 7, characterized in that the coupling reaction is carried out by means of the active ester method.

13. Method according to one of claims 1 to 7, characterized in that used as fusion medium is a buffer solution which contains one of the fusion promoters which is customarily used for the fusion of cells and is selected from the group comprising: sendai viruses or other paramyxoviruses, calcium ions, surface-active lipids or polyethylene glycol.

14. Method according to claim 13, characterized in that said fusion promoters take the form of polyethylene glycol with a mean molecular weight of 600 to 6,000, whose concentration in the fusion medium is 30% - 60%.

15. Method according to one of claims 1 to 7, characterized in that the hybrid cells are cultivated in the presence of isolated macrophages ("feeder cells").

16. Method according to one of claims 1 to 7, characterized in that positive hybrid cell cultures producing monoclonal antibodies are singled out using the limiting dilution method and subsequently cloned in suitable cultivation media.

17. Method according to one of claims 1 to 7, characterized in that the hybridoma cell clones cloned according to claim 16 are investigated using an immunoassay for the formation of suitable monoclonal antibodies.

18. A method for the preparation of mutants and variants of a hybridoma cell line according to one of claims 1 to 7, characterized in that said hybridoma cell line is mutated using methods known per se and those hybridoma cell lines are selected which still have the characteristic properties of the starting material; that is to say are still able to synthesize and to secrete into the surrounding medium the antibodies according to the invention.

19. A method for the preparation of monoclonal antibodies and the derivatives thereof which have a high specificity and affinity for atrazine and which show a pronounced cross-reactivity with structurally similar atrazine derivatives selected from the group comprising propazine, prometryn and prometon but which have essentially no cross-reactivity with other atrazine derivatives, but especially with ametryn and atraton, or with hydroxyl analogues of atrazine and/or atrazine derivatives, selected from the group comprising hydroxyatrazine, hydroxypropazine, hydroxysimazine, hydroxydesmetryn and hydroxyterbuthylazine, characterized in that the hybridoma cell line prepared by a method according to claim 1 is cultivated in vivo or in vitro using known methods, and the produced monoclonal antibodies are isolated.

20. A method according to claim 19, characterized in that the cross-reactivity of said monoclonal antibodies with propazine is more than 80%.

21. A method according to claim 19, characterized in that said hybridoma cell line is the cell line deposited under the number ECACC 8808/2501.

22. A method for the preparation of monoclonal antibodies and the derivatives thereof which have a high specificity and affinity for hydroxyatrazine and which show essentially no cross-reactivity with atrazine and/or atrazine derivatives selected from the group comprising propazine, ametryn and atraton, characterized in that the hybridoma cell line prepared by a method according to claim 3 is cultivated in vivo or in vitro using known methods, and the produced monoclonal antibodies are isolated.

23. A method according to claim 22, characterized in that said monoclonal antibodies have a high specificity and affinity for hydroxyatrazine and show a very pronounced cross-reactivity with hydroxypropazine but show essentially no cross-reactivity with hydroxyl analogues of other atrazine derivatives, selected from the group comprising hydroxysimazine, hydroxydesmetryn and hydroxyterbuthylazine, or with atrazine and/or atrazine derivatives selected from the group comprising propazine, ametryn and atraton.

24. A method according to claim 22, characterized in that said monoclonal antibodies have a high specificity and affinity for hydroxyatrazine and show a cross-reactivity with hydroxyl analogues of atrazine derivatives, selected from the group comprising hydroxypropazine, hydroxysimazine and hydroxydesmetryn, but show essentially no cross-reactivity with atrazine and/or atrazine derivatives selected from the group comprising propazine, ametryn and atraton.

25. A method according to claim 23, characterized in that the cross-reactivity with hydroxypropazine is up to 100%.

26. A method according to claim 24, characterized in that the cross-reactivity with hydroxyl analogues of atrazine derivatives, selected from the group comprising hydroxypropazine, hydroxysimazine and hydroxydesmetryn, is at least 40%.

27. A method according to claim 25, characterized in that said hybridoma cell line is the cell line deposited under the number ECACC 8808/2503.

28. A method according to claim 26, characterized in that said hybridoma cell line is the cell line deposited under the number ECACC 8808/2502.

29. A method according to one of claims 19 to 28, characterized in that said monoclonal antibodies are prepared by expansion in vivo, in a donor animal, of the hybridoma cell lines.

30. A method for the immunological detection of atrazine and/or atrazine derivatives, characterized in that a monoclonal antibody prepared by a method according to one of claims 19 or 20 is used in one of the known immunoassays.

31. A method for the immunological detection of atrazine and/or atrazine derivatives according to claim 30, characterized in that a monoclonal antibody which is produced by a hybridoma cell line prepared by a method according to claim 2, or by clones or subclones thereof, is used in one of the known immunoassays.

32. A method for the immunological detection of hydroxyatrazine and/or hydroxyatrazine derivatives, characterized in that a monoclonal antibody according to one of claims 22 to 26 is used in one of the known immunoassays.

33. A method for the immunological detection of hydroxyatrazine and/or hydroxyatrazine derivatives according to claim 32, characterized in that a monoclonal antibody which is produced by a hybridoma cell line prepared by a method according to claim 6, or by clones or subclones thereof, is used in one of the known immunoassays.

**34.** A method for the immunological detection of hydroxyatrazine and/or hydroxyatrazine derivatives according to claim 32, characterized in that a monoclonal antibody which is produced by a hybridoma cell line prepared by a method according to claim 7, or by clones or subclones thereof, is used in one of the known immunoassays.

**35.** A method according to one of claims 30 to 34, characterized in that it takes the form of a competitive immunoassay.

**36.** A method according to one of claims 30 to 35, characterized in that the said immunoassay takes the form of a radioimmunoassay (RIA), an enzyme-coupled assay (ELISA) or a chemoluminescence assay.

**37.** Means for the immunological detection of atrazine and/or atrazine derivatives in the form of a ready-to-use test kit, characterized in that said test kit contains, besides the customarily used carrier materials, reagents and other additives, at least one monoclonal antibody prepared by a method according to one of claims 19 or 20 as reagent.

**38.** Means for the immunological detection of atrazine and/or atrazine derivatives in the form of a ready-to-use test kit according to claim 37, characterized in that said test kit contains, besides the customarily used carrier materials, reagents and other additives, at least one monoclonal antibody which is produced by a hybridoma cell line prepared by a method according to claim 2, or by clones or subclones thereof.

**39.** Means for the immunological detection of hydroxyatrazine and/or hydroxyl analogues of atrazine derivatives in the form of a ready-to-use test kit, characterized in that said test kit contains, besides the customarily used carrier materials, reagents and other additives, at least one monoclonal antibody prepared by a method according to one of claims 22 to 26 as reagent.

**40.** Means for the immunological detection of hydroxyatrazine and/or hydroxyl analogues of atrazine derivatives in the form of a ready-to-use test kit according to claim 39, characterized in that said test kit contains, besides the customarily used carrier materials, reagents and other additives, at least one monoclonal antibody as reagent which is produced by a hybridoma cell line prepared by a method according to claim 6, or by clones or subclones thereof.

**41.** Means for the immunological detection of hydroxyatrazine and/or hydroxyl analogues of atrazine derivatives in the form of a ready-to-use test kit according to claim 39, characterized in that said test kit contains, besides the customarily used carrier materials, reagents and other additives, at least one monoclonal antibody as reagent which is produced by a hybridoma cell line prepared by a method according to claim 7, or by clones or subclones thereof.

**Revendications**

**Revendications pour les Etats contractants suivants : AT, BE, CH, DE, FR, GB, GR, IT, LI, LU, NL, SE**

**1.** Lignée de cellules d'hybridome qui produit un anticorps monoclonal présentant une spécificité et une affinité élevée vis-à-vis de l'atrazine et qui montre une réactivité croisée marquée avec des dérivés de l'atrazine structurellement semblables choisis parmi le groupe constitué de la propazine, de la prométryne et du prometon, mais qui ne présentent pratiquement aucune réactivité croisée avec d'autres dérivés de l'atrazine, mais en particulier avec l'amétryne et l'atraton, ou avec des analogues hydroxylés de l'atrazine et/ou de dérivés de l'atrazine choisis dans le groupe constitué de l'hydroxyatrazine, de l'hydroxypropazine, de l'hydroxysimazine, de l'hydroxydesmétryne et de l'hydroxyterbuthylazine.

**2.** Lignée de cellules d'hybridome selon la revendication 1, déposée sous le n° ECACC 8808/2501.

**3.** Lignée de cellules d'hybridome qui produit un anticorps monoclonal présentant une spécificité et uné affinité élevées vis-à-vis de l'hydroxyatrazine et qui ne montre pratiquement aucune réactivité croisée avec l'atrazine et/ou les dérivés de l'atrazine, choisis dans le groupe constitué de la propazine, de l'amétryne et de l'atraton.

4. Lignée de cellules d'hybridome selon la revendication 3, qui produit un anticorps monoclonal présentant une spécificité et une affinité élevées vis-à-vis de l'hydroxyatrazine et qui montre une réactivité croisée très marquée avec l'hydroxypropazine, mais qui ne montre pratiquement aucune réactivité croisée avec des analogues hydroxylés d'autres dérivés de l'atrazine choisis dans le groupe constitué de l'hydroxysimazine, de l'hydroxydesmétryne et de l'hydroxyterbutylazine, ou avec l'atrazine et/ou des dérivés de l'atrazine choisis dans le groupe constitué de la propazine, de l'amétryne et de l'atraton.

5. Lignée de cellules d'hybridome selon la revendication 3, qui produit un anticorps monoclonal présentant une spécificité et une affinité élevées vis-à-vis de l'hydroxyatrazine et qui montre une réactivité croisée avec des analogues hydroxylés de dérivés de l'atrazine choisis parmi l'hydroxypropazine, l'hydroxysimazine et l'hydroxydesmétryne, mais qui ne montre pratiquement aucune réactivité croisée avec l'atrazine ou des dérivés de l'atrazine choisis dans le groupe constitué de la propazine, de l'amétryne et de l'atraton.

6. Lignée de cellules d'hybridome selon la revendication 4, déposée sous le n° ECACC 8808/2503.

7. Lignée de cellules d'hybridome selon la revendication 5, déposée sous le n° ECACC 8808/2502.

8. Mutants et variants d'une lignée de cellules d'hybridome selon l'une des revendications 1 à 7, qui présentent encore le spropriétés caractéristiques de la matière de départ ; c'est-à-dire qui sont encore en mesure de synthétiser les anticorps conformes à l'invention et de les secréter dans le milieu environnant.

9. Anticorps monoclonaux et leurs dérivés, qui présentent une spécificité et une affinité élevées vis-à-vis de l'atrazine, et qui montrent une réactivité croisée marquée avec des dérivés de l'atrazine structurellement semblables choisis dans le groupe constitué de la propazine, de la prométryne et du prométon, mais qui ne présentent pratiquement aucune réactivité croisée avec d'autres dérivés de l'atrazine, mais en particulier avec l'amétryne et l'atraton, ou avec des analogues hydroxylés de l'atrazine et/ou de dérivés de l'atrazine choisis dans le groupe constitué de l'hydroxyatrazine, de l'hydroxypropazine, de l'hydroxysimazine, de l'hydroxydesmétryne et de l'hydroxyterbutylazine.

10. Anticorps monoclonaux et leurs dérivés selon la revendication 9, caractérisés en ce que la réactivité croisée avec la propazine est supérieure à 80 %.

11. Anticorps monoclonaux selon la revendication 10, pouvant être obtenus à partir d'une lignée de cellules d'hybridome selon la revendication 2.

12. Anticorps monoclonaux et leurs dérivés, qui présentent une spécificité et une affinité élevées vis-à-vis de l'hydroxyatrazine, et qui ne montrent pratiquement aucune réactivité croisée avec l'atrazine et/ou des dérivés de l'atrazine choisis parmi la propazine, l'amétryne et l'atraton.

13. Anticorps monoclonaux et leurs dérivés selon la revendication 12, qui présentent une spécificité et une affinité élevées vis-à-vis de l'hydroxyatrazine, et qui montrent une réactivité croisée fortement marquée avec l'hydroxypropazine, mais qui ne montre pratiquement aucune réactivité croisée avec des analogues hydroxylés d'autres dérivés de l'atrazine choisis dans le groupe constitué de l'hydroxysimazine, de l'hydroxydesmétryne, et de l'hydroxyterbutylazine, ou avec l'atrazine et/ou des dérivés de l'atrazine choisis dans le groupe constitué de la propazine, de l'amétryne et de l'atraton.

14. Anticorps monoclonaux et leurs dérivés selon la revendication 12, qui présentent une spécificité et une affinité élevées vis-à-vis de l'hydroxyatrazine, et qui montrent une réactivité croisée avec des analogues hydroxylés de dérivés de l'atrazine choisis dans le groupe constitué de l'hydroxypropazine, de l'hydroxysimazine et de l'hydroxydesmétryne, mais qui ne montrent pratiquement aucune réactivité croisée avec l'atrazine et/ou des dérivés de l'atrazine choisis dans le groupe constitué de la propazine, de l'amétryne et de l'atraton.

15. Anticorps monoclonaux et leurs dérivés selon la revendication 13, caractérisés en ce que la réactivité croisée avec l'hydroxypropazine peut aller jusqu'à 100 %.

16. Anticorps monoclonaux et leurs dérivés selon la revendication 14, caractérisés en ce que la réactivité croisée avec des analogues hydroxylés de dérivés de l'atrazine choisis dans le groupe constitué de l'hydroxypropazine, de l'hydroxysimazine et de l'hydroxydesmétryne atteint au moins 40 %.

17. Anticorps monoclonal selon la revendication 15, pouvant être obtenue à partir d'une lignée de cellules d'hybridome selon la revendication 6.

18. Anticorps monoclonal selon la revendication 16, pouvant être obtenu à partir d'une lignée de cellules d'hybridome selon la revendication 7.

19. Procédé de préparation d'une lignée de cellules d'hhybridome selon l'une des revendications 1 à 5, caractérisé en ce que

a) on conjugue l'atrazine et/ou un dérivé de l'atrazine ou l'hydroxyatrazine et/ou un analogue hydroxylé de dérivés de l'atrazine avec une molécule de support ;

b) on immunise un animal donneur avec ce conjugué ;

c) on isole une cellule B immuno-compétente de l'animal donneur immunisé ;

d) on fusionne cette cellule B immuno-compétente avec une lignée de cellules tumorales capables de divisions cellulaires continues ;

e) on isole le produit de fusion formé, on le cultive dans un milieu de culture approprié puis on le clone ; et

f) on étudie les cellules hybrides clonées en ce qui concerne la formation d'anticorps monoclonaux.

20. Procédé selon la revendication 19, caractérisé en ce qu'on utilise comme molécule de support des composés macromoléculaires qui présentent des groupes réactifs librement accessibles pour la réaction de couplage avec l'atrazine et/ou des dérivés de l'atrazine ou l'hydroxyatrzine et/ou des analogues hydroxylés de dérivés de l'atrazine, et qui sont susceptibles de conférer à ces composés un pouvoir immunogène.

21. Procédé selon la revendication 20, caractérisé en ce qu'on utilise une protéine riche en lysine ayant une masse moléculaire comprise entre 10.000 et 1.500.000.

22. procédé selon la revendication 19, caractérisé en ce que la conjugaison à la molécule de support s'effectue par l'intermédiaire d'un élément de pontage (spacer), qui possède un ou plusieurs groupes réactifs capables d'entrer dans une interaction avec des groupes réactifs de la molécule de support.

23. Procédé selon la revendication 22, caractérisé en ce que ces groupes réactifs sont des groupes carboxyle, amino ou SH.

24. Procédé selon la revendication 19, caractérisé en ce que la réaction de couplage est effectuée à l'aide de la méthode aux esters actifs.

25. Procédé selon la revendication 26, caractérisé en ce qu'on utilise comme milieu de fusion une solution tampon qui contient un des promoteurs de fusion habituellement utilisés pour la fusion de cellules, choisi parmi le groupe constitué des virus Sendai ou d'autres paramyxovirus, des ions calcium, des lipides tensio-actifs ou du polyéthylèneglycol.

26. Procédé selon la revendication 25, caractérisé en ce que ces promoteurs de fusion sont un polyéthylèneglycol d'une masse moléculaire de 600 à 6000, dont la concentration dans le milieu de fusion est de 30 % à 60 %.

27. Procédé selon la revendication 19, caractérisé en ce qu'on cultive les cellules hybrides en présence de macrophages isolés ("cellules nourricières").

28. Procédé selon la revendication 19, caractérisé en ce qu'on isole des cultures de cellules hybrides produisant des anticorps monoclonaux à l'aide du procédé de dilution limitative, puis en ce qu'on les clone dans des milieux de culture appropriés.

29. Procédé selon la revendication 19, caractérisé en ce qu'on étudie les clones de cellules d'hybridome, clonées conformément à la revendication 28, à l'aide d'un dosage immunologique, en ce qui concerne la formation d'anticorps monoclonaux appropriés.

30. Procédé de préparation d'anticorps monoclonaux, caractérisé en ce qu'on cultive in vivo ou in vitro les lignées de cellules d'hybridome préparées conformément à un procédé selon l'une des revendications 19 à 29, à l'aide de procédés connus, et en ce qu'on isole les anticorps monoclonaux produits.

EP 0 365 818 B1

**31.** Procédé selon la revendicaiton 30, caractérisé en ce qu'on prépare ces anticorps monoclonaux in vivo, par expansion des lignées de cellules d'hybridome produites par un procédé selon l'une des revendications 19 à 20 dans un animal donneur.

**32.** Procédé de mise en évidence immunologique de l'atrazine et/ou de dérivés de l'atrazine, caractérisé en ce qu'on utilise un anticorps monolconal selon la revendication 9 ou 10 dans un des dosages immunologiques connus.

**33.** Procédé de mise en évidence immunologique de l'atrazine et/ou de dérivés de l'atrazine selon la revendication 2, caractérisé en ce qu'on produit un anticorps monoclonal d'une lignée de cellules d'hybridome selon la revendication 2, ou bien de clones ou de sous-clones de celle-ci dans un dosage immunologique connu.

**34.** Procédé de mise en évidence immunologique de l'hydroxyatrazine et/ou de dérivés de l'hydroxyatrazine, caractérisé en ce qu'on utilise un anticorps monoclonal selon une des revendications 12 à 16, dans un des dosages immunologiques connus.

**35.** Procédé de mise en évidence immunologique de l'hydroxyatrazine et/ou de dérivés de l'hydroxyatrazine selon la revendication 34, caractérisé en ce qu'on utilise un anticorps monoclonal qui est produit par une lignée de cellules d'hybridome selon la revendication 6, ou par des clones ou sous-clones de celle-ci, dans un des dosages immunologiques connus.

**36.** Procédé de mise en évidence immunologique de l'hydroxyatrazine et/ou de dérivés de l'hydroxyatrazine selon la revendication 34, caractérisé en ce qu'on utilise un anticorps monoclonal qui est produit par une lignée de cellules d'hybridome selon la revendication 7, ou par des clones ou sous-clones de celle-ci, dans un des dosages immunologiques connus.

**37.** Procédé selon l'une des revendications 32 à 36, caractérisé en ce qu'il s'agit d'un dosage immunologique compétitif.

**38.** Procédé selon l'une des revendications 32 à 37, caractérisé en ce que ce dosage immunologique est un dosage radio-immunologique (RIA) un dosage par couplage d'enzymes (ELISA) ou un dosage par chimioluminescence.

**39.** Moyen pour la mise en évidence immunologique de l'atrazine et/ou de dérivés de l'atrazine sous la forme d'un nécessaire d'essai prêt à l'emploi, caractérisé en ce que ce nécessaire d'essai contient, outre les matières de support, réactifs et autres additifs habituellement utilisés, au moins un anticorps monoclonal comme réactif selon la revendication 9 ou 10.

**40.** Moyen pour la mise en évidence immunologique de l'atrazine et/ou de dérivés de l'atrazine sous la forme d'un nécessaire d'essai prêt à l'emploi selon la revendication 39, caractérisé en ce que ce nécessaire d'essai contient, outre les matières de support, réactifs et autres additifs habituellement utilisés, au moins un anticorps monoclonal qui est produit par une lignée de cellules d'hybridome selon la revendication 2, ou par des clones ou sous-clones de celle-ci.

**41.** Moyen pour la mise en évidence immunologique de l'hydroxyatrazine et/ou d'analogues de dérivés de l'atrazine sous la forme d'un nécessaire d'essai prêt à l'emploi, caractérisé en ce que ce nécessaire d'essai contient, outre les matières de support, réactifs et autres additifs habituellement utilisés, un anticorps monoclonal selon l'une des revendications 12 à 16.

**42.** Moyen pour la mise en évidence immunologique de l'hydroxyatrazine et/ou d'analogues hydroxylés de dérivés de l'atrazine sous la forme d'un nécessaire d'essai prêt à l'emploi selon la revendication 21, caractérisé en ce que ce nécessaire d'essai contient comme réactif, outre les matières de support, réactifs et autres additifs habituellement utilisés, au moins un anticorps monoclonal qui est produit par une lignée de cellules d'hybridome selon la revendication 6, ou par des clones ou sous-clones de celle-ci.

**43.** Moyen pour la détection immunologique de l'hydroxyatrazine et/ou d'analogues hydroxylés de dérivés de l'atrazine sous la forme d'un nécessaire d'essai prêt à l'emploi selon la revendication 41, caractérisé en ce que ce nécessaire d'essai contient comme réactif, outre les matières de support, réactifs et autres additifs habituellement utilisés, au moins un anticorps monoclonal qui est produit par une lignée de cel-

41

lules d'hybridome selon la revendication 7, ou par des clones ou sous-clones de celle-ci.

**Revendications pour l'Etat contractant suivant : ES**

1. Procédé de préparation d'une lignée de cellules d'hybridome qui produit un anticorps monoclonal présentant une spécificité et une affinité élevées vis-à-vis de l'atrazine, et qui possède une réactivité croisée marquée avec des dérivés d'atrazine structurellement analogues, choisi dans le groupe constitué de la propazine, de la prométryne et du prométon, mais qui ne présente pratiquement aucune réactivité croisée avec d'autres dérivés de l'atrazine, mais en particulier avec l'amétryne et l'atraton, ou avec des analogues hydroxylés de l'atrazine et/ou des dérivés de l'atrazine, choisis dans le groupe constitué de l'hydroxyatrazine, de l'hydroxypropazine, de l'hydroxysimazine, de l'hydroxydesmétryne et de l'hydroxyterbutylazine, caractérisé en ce que
   a) on conjugue l'atrazine et/ou un dérivé de l'atrazine avec une molécule de support ;
   b) on immunise un animal donneur avec ce conjugué ;
   c) on isole une cellule B immuno-compétente de l'animal donneur immunisé ;
   d) on fusionne cette cellule B immuno-compétente avec une lignée de cellules tumorales capables de divisions cellulaires continues ;
   e) on isole le produit de fusion formé, on le cultive dans un milieu de culture approprié puis on le clone ; et
   f) on étudie les cellules hybrides clonées en ce qui concerne la formation d'anticorps monoclonaux.

2. Procédé selon la revendication 1, caractérisé en ce que cette lignée de cellules d'hybridome est une lignée cellulaire déposée sous le n° ECACC 8808/2501.

3. Procédé de préparation d'une lignée de cellules d'hybridome qui produit un anticorps monoclonal présentant une spécificité et une affinité élevées vis-à-vis de l'hydroxyatrazine, et qui ne montre aucune réactivité croisée avec l'atrazine ou avec les dérivés de l'atrazine, choisi dans le groupe constitué de la propazine, de l'amétryne et de l'atraton, caractérisé en ce que
   a) on conjugue l'hydroxyatrazine et/ou des analogues hydroxylés de dérivés de l'atrazine avec une molécule de support ;
   b) on immunise un animal donneur avec ce conjugué ;
   c) on isole une cellule B immuno-compétente de l'animal donneur immunisé ;
   d) on fusionne cette cellule B immuno-compétente avec une lignée de cellules tumorales capables de divisions cellulaires continues ;
   e) on isole le produit de fusion formé, on le cultive dans un milieu de culture approprié puis on le clone ; et
   f) on étudie les cellules hybrides clonées en ce qui concerne la formation d'anticorps monoclonaux.

4. Procédé selon la revendication 3, caractérisé en ce que cette lignée de cellules d'hybridome produit un anticorps monoclonal présentant une spécificité et une affinité élevées vis-à-vis de l'hydroxyatrazine et qui montre une réactivité croisée très marquée avec l'hydroxypropazine, mais qui ne montre pratiquement aucune réactivité croisée avec des analogues hydroxylés d'autres dérivés de l'atrazine choisis dans le groupe constitué de l'hydroxysimazine, de l'hydroxydesmétryne et de l'hydroxyterbutylazine, ou avec l'atrazine et/ou des dérivés de l'atrazine choisis dans le groupe constitué de la propazine, de l'amétryne et de l'atraton.

5. Procédé selon la revendication 3, caractérisé en ce que cette lignée de cellules d'hybridome produit un anticorps monoclonal présentant une spécificité et une affinité élevées vis-à-vis de l'hydroxyatrazine et qui montre une réactivité croisée avec des analogues hydroxylés de dérivés de l'atrazine choisis dans le groupe constitué de l'hydroxypropazine, de l'hydroxysimazine et de l'hydroxydesmétryne, mais qui ne montre pratiquement aucune réactivité croisée avec l'atrazine et/ou des dérivés de l'atrazine choisis dans le groupe constitué de la propazine, de l'amétryne et de l'atraton.

6. Procédé selon la revendication 4, caractérisé en ce que cette lignée de cellules d'hybridome est une lignée cellulaire déposée sous le n° ECACC 8808/2503.

7. Procédé selon la revendication 5, caractérisé en ce cette lignée de cellules d'hybridome est une lignée cellulaire déposée sous le n° ECACC 8808/2502.

8. Procédé selon l'une des revendications 1 à 7, caractérisé en ce qu'on utilise comme molécule de support des composés macromoléculaires qui sont des groupes réactifs librement accessibles pour la réaction de couplage avec l'atrazine et/ou des dérivés de l'atrazine ou l'hydroxyatrzine et/ou des analogues hydroxylés de dérivés de l'atrazine, et qui sont susceptibles de conférer à ces composés un pouvoir immunogène.

9. Procédé selon la revendication 8, caractérisé en ce qu'on utilise une protéine riche en lysine ayant une masse moléculaire comprise entre 10.000 et 1.500.000.

10. procédé selon l'une des revendications 1 à 7, caractérisé en ce que la conjugaison sur la molécule de support s'effectue par l'intermédiaire d'un élément de pontage (spacer), qui possède un ou plusieurs groupes réactifs capables d'entrer dans une interaction avec des groupes réactifs de la molécule de support.

11. Procédé selon la revendication 10, caractérisé en ce que ces groupes réactifs sont des groupes carboxyle, amino ou SH.

12. Procédé selon l'une des revendications 1 à 7, caractérisé en ce que la réaction de couplage est effectuée à l'aide de la méthode aux esters actifs.

13. Procédé selon l'une des revendications 1 à 7, caractérisé en ce qu'on utilise comme milieu de fusion une solution tampon qui contient des promoteurs de fusion habituellement utilisés pour la fusion des cellules, choisi dans le groupe constitué des virus Sendai et d'autres paramyxovirus, des ions calcium, des lipides tensio-actifs ou du polyéthylèneglycol.

14. Procédé selon la revendication 13, caractérisé en ce que ces promoteurs de fusion sont un polyéthylèneglycol d'une masse moléculaire de 600 à 6000, dont la concentration dans le milieu de fusion est de 30 % à 60 %.

15. Procédé selon l'une des revendications 1 à 7, caractérisé en ce qu'on cultive les cellules hybrides en présence de macrophages isolés ("cellules nourricières").

16. Procédé selon l'une des revendications 1 à 7, caractérisé en ce qu'on isole des cultures de cellules hybrides produisant des anticorps monoclonaux à l'aide du procédé de dilution limitative, puis en ce qu'on les clone dans des milieux de culture appropriés.

17. Procédé selon l'une des revendications 1 à 7, caractérisé en ce qu'on étudie les clones de cellules d'hybridome, clonées conformément à la revendication 28, à l'aide d'un dosage immunologique, en ce qui concerne la formation d'anticorps monoclonaux appropriés.

18. Procédé de préparation de mutants et de variants d'une lignée de cellules d'hybridome selon l'une des revendications 1 à 7, caractérisé en ce qu'on effectue une mutation de cette lignée de cellules d'hybridome à l'aide de procédés connus en soi et qu'on sélectionne les lignées de cellules d'hybridome qui présentent encore les propriétés caractéristiques de la matière de départ c'est-à-dire qui sont encore en mesure de synthétiser les anticorps conformes à l'invention et de les secréter dans le milieu environnant.

19. Procédé de préparation d'anticorps monoclonaux et de leurs dérivés qui présente une spécificité et une affinité élevées vis-à-vis de l'atrazine, et qui montre une réactivité croisée marquée avec des dérivés d'atrazine structurellement analogues, du groupe constitué de la propazine, de la prométryne et du prométon, mais qui ne présente pratiquement aucune réactivité croisée avec d'autres dérivés de l'atrazine, mais en particulier avec l'amétryne et l'atraton, ou avec des analogues hydroxylés de l'atrazine et/ou des dérivés de l'atrazine, choisis dans le groupe constitué de l'hydroxyatrazine, de l'hydroxypropazine, de l'hydroxysimazine, de l'hydroxydesmétryne et de l'hydroxyterbutylazine, caractérisé en ce qu'on cultive in vivo ou in vitro les lignées de cellules d'hybridome préparées par un procédé selon la revendication 1, à l'aide de procédés connus, et on isole les anticorps monoclonaux produits.

20. Procédé selon la revendication 19, caractérisé en ce que la réactivité croisée de ces anticorps monoclonaux avec la propazine est supérieure à 80 %.

21. Procédé selon la revendication 19, caractérisé en ce que cette lignée de cellules d'hybridome est une lignée cellulaire déposée sous le n° ECACC 8808/2501.

**22.** Procédé de préparation d'anticorps monoclonaux et de leurs dérivés qui présente une spécificité et une affinité élevées vis-à-vis de l'hydroxyatrazine, et qui ne montre pratiquement aucune réactivité croisée avec l'atrazine et/ou des dérivés de l'atrazine, choisi parmi la propazine, l'amétryne et l'atraton, caractérisé en ce qu'on cultive la lignée de cellules d'hybridome préparée par un procédé selon la revendication 3, in vivo ou in vitro, à l'aide de procédés connus, et en ce qu'on isole les anticorps monoclonaux produits.

**23.** Procédé selon la revendication 22, caractérisé en ce que ces anticorps monoclonaux présentent une spécificité et une affinité élevées vis-à-vis de l'hydroxyatrazine et montrent une réactivité croisée marquée avec l'hydroxypropazine, mais pratiquement aucune réactivité croisée avec des analogues hydroxylés d'autres dérivés de l'atrazine, choisis dans le groupe constitué de l'hydroxysimazine, de l'hydroxydesmétryne et de l'hydroxyterbutylazine, ou avec l'atrazine et/ou des dérivés de l'atrazine choisis dans le groupe constitué de la propazine, de l'amétryne et de l'atraton.

**24.** Procédé selon la revendication 22, caractérisé en ce que ces anticorps monoclonaux présentent une spécificité et une affinité élevées vis-à-vis de l'hydroxyatrazine et une réactivité croisée avec des analogues hydroxylés de dérivés de l'atrazine choisis dans le groupe constitué de l'hydroxypropazine, l'hydroxysimazine et l'hydroxydesmétryne, mais pratiquement aucune réactivité croisée avec l'atrazine et/ou des dérivés de l'atrazine, choisi dans le groupe constitué de la propazine, de l'amétryne et de l'atraton.

**25.** Procédé selon la revendication 23, caractérisé en ce que la réactivité croisée avec l'hydroxypropazine atteint une valeur de 100 %.

**26.** Procédé selon la revendication 24, caractérisé en ce que la réactivité croisée avec des analogues hydroxylés de dérivés de l'atrazine choisis parmi l'hydroxypropazine, l'hydroxysimazine et l'hydroxydesmétryne, est d'au moins 40 %.

**27.** Procédé selon la revendication 25, caractérisé en ce que cette lignée de cellules d'hybridome est une lignée cellulaire déposée sous n° ECACC/2503.

**28.** Procédé selon la revendication 26, caractérisé en ce que cette lignée de cellules d'hybridome est une lignée cellulaire déposée sous le n° ECACC 8808/2502.

**29.** Procédé selon l'une des revendications 19 à 28, caractérisé en ce qu'on prépare ces anticorps monoclonaux par expansion des lignées de cellules d'hybridome dans un animal donneur, in vivo.

**30.** Procédé de mise en évidence immunologique de l'atrazine et/ou de dérivés de l'atrazine, caractérisé en ce qu'on utilise un anticorps monoclonal préparé par un procédé selon la revendication 19 ou 19 dans un des dosages immunologiques connus.

**31.** Procédé de mise en évidence immunologique de l'atrazine et/ou de dérivés de l'atrazine selon la revendication 30, caractérisé en ce qu'on utilise un anticorps monoclonal qui est produit par une lignée de cellules d'hybridome préparé par le procédé selon la revendication 2, ou des clones ou sous-clones de celle-ci dans un des dosages immunologiques connus.

**32.** Procédé de mise en évidence immunologique de l'hydroxyatrazine et/ou de dérivés de l'hydroxyatrazine, caractérisé en ce qu'on utilise un anticorps monoclonal selon une des revendications 22 à 26, dans un des dosages immunologiques connus.

**33.** Procédé de mise en évidence immunologique de l'hydroxyatrazine et/ou de dérivés de l'hydroxyatrazine selon la revendication 32, caractérisé en ce qu'on utilise un anticorps monoclonal qui est produit par une lignée de cellules d'hybridome préparée par un procédé selon la revendication 6, ou par des clones ou sous-clones de celle-ci, dans un des dosages immunologiques connus.

**34.** Procédé de mise en évidence immunologique de l'hydroxyatrazine et/ou de dérivés de l'hydroxyatrazine selon la revendication 32, caractérisé en ce qu'on utilise un anticorps monoclonal qui est produit par une lignée de cellules d'hybridome préparée par un procédé selon la revendication 7, ou par des clones ou sous-clones de celle-ci, dans un des dosages immunologiques connus.

**35.** Procédé selon l'une des revendications 30 à 34, caractérisé en ce qu'il s'agit d'un dosage immunologique compétitif.

36. Procédé selon l'une des revendications 30 à 35, caractérisé en ce que ce dosage immunologique est un dosage radio-immunologique (RIA) un dosage par couplage d'enzymes (ELISA) ou un dosage par chimioluminescence.

37. Moyen pour la mise en évidence immunologique de l'atrazine et/ou de dérivés de l'atrazine sous la forme d'un nécessaire d'essai prêt à l'emploi, caractérisé en ce que ce nécessaire d'essai contient comme réactif, outre les matières de support, réactifs et autres additifs habituellement utilisés, au moins un anticorps monoclonal préparé par un procédé selon la revendication 19 ou 20.

38. Moyen pour la mise en évidence immunologique de l'atrazine et/ou de dérivés de l'atrazine sous la forme d'un nécessaire d'essai prêt à l'emploi selon la revendication 37, caractérisé en ce que ce nécessaire d'essai contient, outre les matières d'essai, réactifs et autres additifs habituellement utilisés, au moins un anticorps monoclonal qui est produit par une lignée de cellules d'hybridome, préparée par un procédé selon la revendication 2, ou par des clones ou sous-clones de celle-ci.

39. Moyen pour la mise en évidence immunologique de l'hydroxyatrazine et/ou d'analogues hydroxylés de dérivés de l'atrazine sous la forme d'un nécessaire d'essai prêt à l'emploi, caractérisé en ce que ce nécessaire d'essai contient comme réactif, outre les matières d'essai, réactifs et autres additifs habituellement utilisés, au moins un anticorps monoclonal préparé par un procédé selon l'une des revendications 22 à 26.

40. Moyen pour la mise en évidence immunologique de l'hydroxyatrazine et/ou d'analogues hydroxylés de dérivés de l'atrazine sous la forme d'un nécessaire d'essai prêt à l'emploi selon la revendication 39, caractérisé en ce que ce nécessaire d'essai contient comme réactif, outre les matières de support, réactifs et autres additifs habituellement utilisés, au moins un anticorps monoclonal qui est produit par une lignée de cellules d'hybridome préparée par un procédé selon la revendication 6, ou par des clones ou sous-clones de celle-ci.

41. Moyen pour la détection immunologique de l'hydroxyatrazine et/ou d'analogues hydroxylés de dérivés de l'atrazine sous la forme d'un nécessaire d'essai prêt à l'emploi selon la revendication 39, caractérisé en ce que ce nécessaire d'essai contient comme réactif, outre les matières de support, réactifs et autres additifs habituellement utilisés, au moins un anticorps monoclonal qui est produit par une lignée de cellules d'hybridome préparée par un procédé selon la revendication 7, ou par des clones ou sous-clones de celle-ci.